# EUROPEAN PATENT APPLICATION

(11) **EP 4 180 456 A1**
(43) Date of publication of application: **17.05.2023**
(21) Application number: 21838742.1
(22) Date of filing: 09.07.2021
(51) Int. Cl.: C07K 16/08, C07K 16/00, C12N 15/13, A61P 31/20, A61K 39/395

(54) **ANTIBODY BINDING TO HEPATITIS B VIRUS SURFACE ANTIGEN AND APPLICATION OF ANTIBODY**

(30) Priority: 09.07.2020 CN 202010659026; 10.07.2020 CN 202010659828
(71) Applicant: Beijing Kawin Technology Share-Holding Co., Ltd., Beijing 100176 (CN)
(72) Inventor: XU, Zheng, Beijing 100176 (CN); CHANG, Hongyan, Beijing 100176 (CN); LI, Xiang, Beijing 100176 (CN); SONG, Rui, Beijing 100176 (CN); LIU, Ying, Beijing 100176 (CN); LI, Feng, Beijing 100176 (CN)
(74) Representative: Lavoix
(86) International application number: PCT/CN2021/105523
(87) International publication number: WO 2022/007955

(57) **Abstract**

Provided are an antibody or antigen-binding fragment thereof that specifically binds to hepatitis B virus surface antigen (HBsAg), a pharmaceutical composition containing the antibody or antigen-binding fragment, and use thereof. Further provided are a nucleic acid molecule encoding the antibody, a vector and a host cell containing the nucleic acid molecule, and a method for preparing the antibody.

## Description

### CROSS REFERENCES TO RELATED APPLICATIONS

This application claims the priority of Chinese application 202010659026.2 filed on July 9, 2020, and Chinese application 202010659828.3 filed on July 10, 2020, the entire contents of which are hereby incorporated by reference.

### TECHNICAL FIELD

The application belongs to the technical field of antibodies, and relates to an antibody binding to hepatitis B virus surface antigen and the use of the antibody in treating diseases related to hepatitis B virus infection.

### BACKGROUND ART

Hepatitis B virus (HBV) infection is prevalent worldwide, but the epidemic intensity of HBV infection varies greatly in different regions. According to the report of the World Health Organization, about 2 billion people in the world have been infected with HBV, of which 240 million people are chronically infected with HBV, and about 650,000 people die of liver failure, cirrhosis and hepatocellular carcinoma (HCC) caused by HBV infection every year. HBV infection accounts for 30% and 45% of global liver cirrhosis and HCC patients, respectively. HBV infection accounts for 60% and 80% of patients with liver cirrhosis and HCC in China, respectively. China is a country with a large population of chronic hepatitis B, and epidemiological research shows that more than 7% of people are infected with hepatitis B virus, and the total number is close to 100 million. By the end of 2015, only 9% of people with HBV infection had been tested and diagnosed; only 8% of those diagnosed with HBV infection had received treatment (data from the China Hepatitis Prevention Foundation). Not only do they require long-term or even life-long treatment, which brings a huge burden to the family and society, but they also have the risk of developing liver cirrhosis and liver cancer. According to estimates, there are 28 million chronic hepatitis B patients in China, and nearly one million liver cirrhosis patients and about 300,000 liver cancer patients are newly added every year. According to the visiting rate of 5% of patients with chronic hepatitis and 95% of patients with liver cirrhosis and liver cancer, the direct medical expenses related to the treatment of hepatitis B in China reach 80 billion to 120 billion yuan per year.

After antiviral treatment in patients with hepatitis B, although the viral load in the patients can be reduced and the progression of severe disease can be delayed, many people cannot completely eliminate the hepatitis B virus. Patients with hepatitis B need to take medicine for a long time, and the financial burden on patients is serious. According to the data dynamically released by Chinese Center for Disease Control and Prevention, the average annual diagnosis and treatment expenses of chronic hepatitis patients accounted for 56.24% of the average annual family income, 81.53% for compensated cirrhosis, 157.21% for decompensated cirrhosis, and 96.76% for liver cancer. The heavy economic burden causes many patients to either give up treatment or causes their families to return to poverty due to illness. In addition, judging from the evolution of liver disease diagnosis and treatment expenses in the past ten years, the diagnosis and treatment expenses of liver diseases show a trend of increasing year by year, with an annual increase of about 10% to 20%, and the part borne by individuals also increases accordingly.

Studies have shown that a large number of subviral particles composed of HBsAg in the blood of patients with hepatitis B are the main reason for suppression of the immune system and the inability to remove infected liver cells. Reducing the level of blood HBsAg by 10-100 times through a certain technology can activate the body's own T cell immunity, so that the body's own immune system can begin to effectively kill the infected liver cells. Therefore, effectively reducing the level of blood HBsAg is not only one of the important criteria for curing hepatitis B, but also a prerequisite for treating hepatitis B to remove infected cells.

The latest clinical research results show that for patients who have been treated with nucleotide analogs to clear viral DNA, if HBsAg is lower than a certain level (such as HBsAg < 1500 IU/ml), the probability of interferon to clear HBsAg can exceed 50%, which is much higher than the cure ratio of 5-10% of interferon that we usually think.

However, due to the lack of drugs that can effectively reduce HBsAg, the HBsAg level of most patients (more than 90%) exceeds this index, resulting in the inability of interferon to cure these patients (this is why the cure rate of interferon is only 5-10%). These new clinical evidences show a more realistic and urgent need for HBsAg-lowering treatments.

Current studies have found that there are three main ways to reduce hepatitis B surface antigen (HBsAg): inhibiting the expression of HBsAg (siRNA), preventing the release of HBsAg from cells (NAPs), and HBsAg antibodies.

The research direction of using antibody technology to reduce HBsAg level has been determined. After screening, we have obtained multiple strains of antibodies. In vitro studies have shown that the antibody of the present application can specifically bind to HBsAg. The results of animal model studies have shown that it can effectively reduce HBsAg levels.

### SUMMARY OF THE APPLICATION

One of the purposes of the present application is to provide an antibody or an antigen-binding fragment thereof that specifically binds to hepatitis B virus surface antigen (HBsAg). The in vitro antigen affinity test proves that the antibody of the present application has the ability to specifically bind HBsAg. The in vivo test further proves that the antibody of the present application can be used as a neutralizing antibody for hepatitis B virus (HBV), and effectively reducing the level of HBsAg and inhibiting the proliferation of HBV

Specifically, this application relates to:
1. An antibody or an antigen-binding fragment thereof specifically binding to hepatitis B virus surface antigen (HBsAg), comprising a heavy chain variable region (VH) sequence and a light chain variable region (VL) sequence, wherein:
the heavy chain variable region comprises complementarity determining region (CDR) amino acid sequences as shown below:
   HCDR1: X1YX3FX5X6X7Y (SEQ ID NO: 33),
   HCDR2: X11NX13X14X15X16X17X18 (SEQ ID NO: 34),
   and HCDR3: ARDX21WX23X24X25X26DX28YGMDX33 (SEQ ID NO: 35);
the light chain variable region comprises the CDR amino acid sequences as shown below:
   LCDR1: X34X35X36SX38X39 (SEQ ID NO: 36),
   LCDR2: X40X41X42 (SEQ ID NO: 37),
   and LCDR3: QQSYSTPLX51 (SEQ ID NO: 38);
wherein X1, X3, X5, X6, X7, X11, X13, X14, X15, X16, X17, X18, X21, X23, X24, X25, X26, X28, X33, X34, X35, X36, X38, X39, X40, X41, X42, and X51 are each selected from any amino acid.

2. The antibody or antigen-binding fragment thereof specifically binding to HBsAg according to item 1, wherein:
X1 is selected from G or A; X3 is selected from T, A or S; X5 is selected from T, A or I; X6 is selected from G, A, Y or D, X7 is selected from Y or A, X11 is selected from I or A, X13 is selected from P or A, X14 is selected from N, A or Y, X15 is selected from S, A or N, X16 is selected from G or A, X17 is selected from G or A, X18 is selected from T or A, X21 is selected from L, V or A, X23 is selected from N, Q or A, X24 is selected from D, Q or A, X25 is selected from D, G or A, X26 is selected from V, G or A, X28 is selected from Y or A, X33 is selected from V or A; X34 is selected from Q or A; X35 is selected from S or A; X36 is selected from I, A or V, X38 is selected from T, A or S, X39 is selected from Y or A, X40 is selected from A, G, D, T or S, X41 is selected from A or S, X42 is selected from A or S, and X51 is selected from T or A.
3. The antibody or antigen-binding fragment thereof specifically binding to HBsAg according to item 2, wherein:
X1, X3, X5, X6 and X7 in HCDR1 are successively selected from any combination of the following:
G, T, T, G, Y; A, T, T, G, Y; G, A, T, G, Y; G, T, A, G, Y; G, T, T, A, Y; G, T, T, G, A; G, S, I, G, Y; G, T, T, D, Y; G, T, T, Y, Y;
X11, X13, X14, X15, X16, X17 and X18 in HCDR2 are successively selected from any combination of the following: I, P, N, S, G, G, T; A, P, N, S, G, G, T; I, A, N, S, G, G, T; I, P, A, S, G, G, T; I, P, N, A, G, G, T; I, P, N, S, A, G, T; I, P, N, S, G, A, T; I, P, N, S, G, G, A; I, P, Y, N, G, G, T,
X21, X23, X24, X25, X26, X28 and X33 in HCDR3 are successively selected from any combination of the following: L, N, D, D, V, Y, V; A, N, D, D, V, Y, V; L, A, D, D, V, Y, V; L, N, A, D, V, Y, V; L, N, D, A, V, Y, V; L, N, D, D, A, Y, V; L, N, D, D, V, A, V; L, N, D, D, V, Y, A; V, Q, Q, G, G, Y, V;
X34, X35, X36, X38 and X39 in LCDR1 are successively selected from any combination of the following: Q, S, I, T, Y; A, S, I, T, Y; Q, A, I, T, Y; Q, S, A, T, Y; Q, S, I, A, Y; Q, S, I, T, A; Q, S, I, S, Y; Q, S, V, S, Y;
X40, X41, and X42 in LCDR2 are successively selected from any combination of the following: A, A, S; S, A, S; A, S, S; A, A, A; G, A, S; D, A, S; T, A, S; and
X51 in LCDR3 is selected from: T or A.

4. The antibody or antigen-binding fragment thereof specifically binding to HBsAg according to item 3, wherein X1, X3, X5, X6, X7, X11, X13, X14, X15, X16, X17, X18, X21, X23, X24, X25, X26, X28 and X33 are successively selected from any combination in Table 1 below:

**Table 1:**

| Antibody name | HCDR1 | HCDR2 | HCDR3 |
|---|---|---|---|
| 021/005/0 62/079/09 5/096 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V |
| 083 | X1 is G, X3 is T, X5 is T, X6 is Y, X7 is Y | X11 is I, X13 is P, X14 is N, X15 is S, X16 is G, X17 is G, X18 is T | X21 is V, X23 is Q, X24 is Q, X25 is G, X26 is G, X28 is Y, X33 is V |
| 021-M1 | X1 is A, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V |
| 021-M3 | X1 is G, X3 is A, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V |
| 021-M5 | X1 is G, X3 is T, X5 is A, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V |
| 021-M6 | X1 is G, X3 is T, X5 is T, X6 is A, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V |
| 021-M7 | X1 is G, X3 is T, X5 is T, X6 is G, X7 is A | X11 is I, X13 is P, X14 is N, X15 is S, X16 is G, X17 is G, X18 is T | X21 is L, X23 is N, X24 is D, X25 is D, X26 is V, X28 is Y, X33 is V |
| 021-M11 | X1 is G, X3 is T, X5 is T, X6 is G, X7 is Y | X11 is A, X13 is P, X14 is N, X15 is S, X16 is G, X17 is G, X18 is T | X21 is L, X23 is N, X24 is D, X25 is D, X26 is V, X28 is Y, X33 is V |
| 021-M13 | X1 is G, X3 is T, X5 is T, X6 is G, X7 is Y | X11 is I, X13 is A, X14 is N, X15 is S, X16 is G, X17 is G, X18 is T | X21 is L, X23 is N, X24 is D, X25 is D, X26 is V, X28 is Y, X33 is V |
| 021-M14 | X1 is G, X3 is T, X5 is T, X6 is G, X7 is Y | X11 is I, X13 is P, X14 is A, X15 is S, X16 is G, X17 is G, X18 is T | X21 is L, X23 is N, X24 is D, X25 is D, X26 is V, X28 is Y, X33 is V |
| 021-M15 | X1 is G, X3 is T, X5 is T, X6 is G, X7 is Y | X11 is I, X13 is P, X14 is N, X15 is A, X16 is G, X17 is G, X18 is T | X21 is L, X23 is N, X24 is D, X25 is D, X26 is V, X28 is Y, X33 is V |
| 021-M16 | X1 is G, X3 is T, X5 is T, X6 is G, X7 is Y | X11 is I, X13 is P, X14 is N, X15 is S, X16 is A, X17 is G, X18 is T | X21 is L, X23 is N, X24 is D, X25 is D, X26 is V, X28 is Y, X33 is V |
| 021-M17 | X1 is G, X3 is T, X5 is T, X6 is G, X7 is Y | X11 is I, X13 is P, X14 is N, X15 is S, X16 is G, X17 is A, X18 is T | X21 is L, X23 is N, X24 is D, X25 is D, X26 is V, X28 is Y, X33 is V |
| 021-M18 | X1 is G, X3 is T, X5 is T, X6 is G, X7 is Y | X11 is I, X13 is P, X14 is N, X15 is S, X16 is G, X17 is G, X18 is A | X21 is L, X23 is N, X24 is D, X25 is D, X26 is V, X28 is Y, X33 is V |
| 021-M21 | X1 is G, X3 is T, X5 is T, X6 is G, X7 is Y | X11 is I, X13 is P, X14 is N, X15 is S, X16 is G, X17 is G, X18 is T | X21 is A, X23 is N, X24 is D, X25 is D, X26 is V, X28 is Y, X33 is V |
| 021-M23 | X1 is G, X3 is T, X5 is T, X6 is G, X7 is Y | X11 is I, X13 is P, X14 is N, X15 is S, X16 is G, X17 is G, X18 is T | X21 is L, X23 is A, X24 is D, X25 is D, X26 is V, X28 is Y, X33 is V |
| 021-M24 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is A, X25 is D, X26 is V, X28 is Y, X33 is V |
| 021-M25 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is A, X26 is V, X28 is Y, X33 is V |
| 021-M26 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is A, X28 is Y, X33 is V |
| 021-M28 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is A, X33 is V |
| 021-M33 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is A |
| 088 | X1 is G, X3 is S, X5 is I, X6 is G, X7 is Y | X11 is I, X13 is P, X14 is Y, X15 is N, X16 is G, X17 is G, X18 is T | X21 is L, X23 is N, X24 is D, X25 is D, X26 is V, X28 is Y, X33 is V |
| 090/091/0 93 | X1 is G, X3 is T, X5 is T, X6 is D, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V |

5. The antibody or antigen-binding fragment thereof specifically binding to HBsAg according to item 3 or 4, wherein X34, X35, X36, X38, X39, X40, X41, X42 and X51 are successively selected from any combination in Table 2 below:

**Table 2:**

| Antibody name | LCDR1 | LCDR2 | LCDR3 |
|---|---|---|---|
| 021/083 | X34 is Q, X35 is S, X36 is I, X38 is T, X39 is Y | X40 is A, X41 is A, X42 is S | X51 is T |
| 021-M34 | X34 is A, X35 is S, X36 is I, X38 is T, X39 is Y | X40 is A, X41 is A, X42 is S | X51 is T |
| 021-M35 | X34 is Q, X35 is A, X36 is I, X38 is T, X39 is Y | X40 is A, X41 is A, X42 is S | X51 is T |
| 021-M36 | X34 is Q, X35 is S, X36 is A, X38 is T, X39 is Y | X40 is A, X41 is A, X42 is S | X51 is T |
| 021-M38 | X34 is Q, X35 is S, X36 is I, X38 is A, X39 is Y | X40 is A, X41 is A, X42 is S | X51 is T |
| 021-M39 | X34 is Q, X35 is S, X36 is I, X38 is T, X39 is A | X40 is A, X41 is A, X42 is S | X51 is T |
| 021-M40 | X34 is Q, X35 is S, X36 is I, X38 is T, X39 is Y | X40 is S, X41 is A, X42 is S | X51 is T |
| 021-M41 | X34 is Q, X35 is S, X36 is I, X38 is T, X39 is Y | X40 is A, X41 is S, X42 is S | X51 is T |
| 021-M42 | X34 is Q, X35 is S, X36 is I, X38 is T, X39 is Y | X40 is A, X41 is A, X42 is A | X51 is T |
| 021-M51 | X34 is Q, X35 is S, X36 is I, X38 is T, X39 is Y | X40 is A, X41 is A, X42 is S | X51 is A |
| 005/062/0 79/088/09 5 | X34 is Q, X35 is S, X36 is I, X38 is S, X39 is Y | X40 is A, X41 is A, X42 is S | X51 is T |
| 090 | X34 is Q, X35 is S, X36 is V, X38 is S, X39 is Y | X40 is G, X41 is A, X42 is S | X51 is T |
| 091 | X34 is Q, X35 is S, X36 is V, X38 is S, X39 is Y | X40 is D, X41 is A, X42 is S | X51 is T |
| 093 | X34 is Q, X35 is S, X36 is I, X38 is S, X39 is Y | X40 is D, X41 is A, X42 is S | X51 is T |
| 096 | X34 is Q, X35 is S, X36 is I, X38 is S, X39 is Y | X40 is T, X41 is A, X42 is S | X51 is T |

6. The antibody or antigen-binding fragment thereof specifically binding to HBsAg according to item 3, wherein X1, X3, X5, X6, X7, X11, X13, X14, X15, X16, X17, X18, X21, X23, X24, X25, X26, X28, X33, X34, X35, X36, X38, X39, X40, X41, X42 and X51 are successively selected from any combination in the following table 3:

**Table 3:**

| anti bod Y | HCDR1 | HCDR2 | HCDR3 | LCDR1 | LCDR2 | LCD R3 |
|---|---|---|---|---|---|---|
| 021 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is T, X39 is Y | X40 is A, X41 is A, X42 is S | X51 is T |
| 021 -M 1 | X1 is A, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is T, X39 is Y | X40 is A, X41 is A, X42 is S | X51 is T |
| 021 -M 3 | X1 is G, X3 is A, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is T, X39 is Y | X40 is A, X41 is A, X42 is S | X51 is T |
| 021 -M 5 | X1 is G, X3 is T, X5 is A, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is T, X39 is Y | X40 is A, X41 is A, X42 is S | X51 is T |
| 021 -M 6 | X1 is G, X3 is T, X5 is T, X6 is A, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is T, X39 is Y | X40 is A, X41 is A, X42 is S | X51 is T |
| 021 -M 7 | X1 is G, X3 is T, X5 is T, X6 is G, X7 is A | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is T, X39 is Y | X40 is A, X41 is A, X42 is S | X51 is T |
| 021 -M 11 | X1 is G, X3 is T, X5 is T, X6 is G, X7 is Y | X11 is A, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is T, X39 is Y | X40 is A, X41 is A, X42 is S | X51 is T |
| 021 -M 13 | X1 is G, X3 is T, X5 is T, X6 is G, X7 is Y | X11 is I, X13 is A, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is T, X39 is Y | X40 is A, X41 is A, X42 is S | X51 is T |
| 021 -M 14 | X1 is G, X3 is T, X5 is T, X6 is G, X7 is Y | X11 is I, X13 is P, X14 is A, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is T, X39 is Y | X40 is A, X41 is A, X42 is S | X51 is T |
| 021 -M 15 | X1 is G, X3 is T, X5 is T, X6 is G, X7 is Y | X11 is I, X13 is P, X14 is N, X15 is A, X16 is G, X17 is G, X18 is T | X21 is L, X23 is N, X24 is D, X25 is D, X26 is V, X28 is Y, X33 is V | X34 is Q, X35 is S, X36 is I, X38 is T, X39 is Y | X40 is A, X41 is A, X42 is S | X51 is T |
| 021 -M 16 | X1 is G, X3 is T, X5 is T, X6 is G, X7 is Y | X11 is I, X13 is P, X14 is N, X15 is S, X16 is A, X17 is G, X18 is T | X21 is L, X23 is N, X24 is D, X25 is D, X26 is V, X28 is Y, X33 is V | X34 is Q, X35 is S, X36 is I, X38 is T, X39 is Y | X40 is A, X41 is A, X42 is S | X51 is T |
| 021 -M 17 | X1 is G, X3 is T, X5 is T, X6 is G, X7 is Y | X11 is I, X13 is P, X14 is N, X15 is S, X16 is G, X17 is A, X18 is T | X21 is L, X23 is N, X24 is D, X25 is D, X26 is V, X28 is Y, X33 is V | X34 is Q, X35 is S, X36 is I, X38 is T, X39 is Y | X40 is A, X41 is A, X42 is S | X51 is T |
| 021 -M 18 | X1 is G, X3 is T, X5 is T, X6 is G, X7 is Y | X11 is I, X13 is P, X14 is N, X15 is S, X16 is G, X17 is G, X18 is A | X21 is L, X23 is N, X24 is D, X25 is D, X26 is V, X28 is Y, X33 is V | X34 is Q, X35 is S, X36 is I, X38 is T, X39 is Y | X40 is A, X41 is A, X42 is S | X51 is T |
| 021 -M 21 | X1 is G, X3 is T, X5 is T, X6 is G, X7 is Y | X11 is I, X13 is P, X14 is N, X15 is S, X16 is G, X17 is G, X18 is T | X21 is A, X23 is N, X24 is D, X25 is D, X26 is V, X28 is Y, X33 is V | X34 is Q, X35 is S, X36 is I, X38 is T, X39 is Y | X40 is A, X41 is A, X42 is S | X51 is T |
| 021 -M 23 | X1 is G, X3 is T, X5 is T, X6 is G, X7 is Y | X11 is I, X13 is P, X14 is N, X15 is S, X16 is G, X17 is G, X18 is T | X21 is L, X23 is A, X24 is D, X25 is D, X26 is V, X28 is Y, X33 is V | X34 is Q, X35 is S, X36 is I, X38 is T, X39 is Y | X40 is A, X41 is A, X42 is S | X51 is T |
| 021 -M 24 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is A, X25 is D, X26 is V, X28 is Y, X33 is V | X34 is Q, X35 is S, X36 is I, X38 is T, X39 is Y | X40 is A, X41 is A, X42 is S | X51 is T |
| 021 -M 25 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is A, X26 is V, X28 is Y, X33 is V | X34 is Q, X35 is S, X36 is I, X38 is T, X39 is Y | X40 is A, X41 is A, X42 is S | X51 is T |
| 021 -M 26 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is A, X28 is Y, X33 is V | X34 is Q, X35 is S, X36 is I, X38 is T, X39 is Y | X40 is A, X41 is A, X42 is S | X51 is T |
| 021 -M 28 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is A, X33 is V | X34 is Q, X35 is S, X36 is I, X38 is T, X39 is Y | X40 is A, X41 is A, X42 is S | X51 is T |
| 021 -M 33 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is A | X34 is Q, X35 is S, X36 is I, X38 is T, X39 is Y | X40 is A, X41 is A, X42 is S | X51 is T |
| 021 -M 34 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is A, X35 is S, X36 is I, X38 is T, X39 is Y | X40 is A, X41 is A, X42 is S | X51 is T |
| 021 -M 35 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is A, X36 is I, X38 is T, X39 is Y | X40 is A, X41 is A, X42 is S | X51 is T |
| 021 -M 36 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is A, X38 is T, X39 is Y | X40 is A, X41 is A, X42 is S | X51 is T |
| 021 -M 38 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is A, X39 is Y | X40 is A, X41 is A, X42 is S | X51 is T |
| 021 -M 39 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is T, X39 is A | X40 is A, X41 is A, X42 is S | X51 is T |
| 021 -M 40 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is T, X39 is Y | X40 is S, X41 is A, X42 is S | X51 is T |
| 021 -M 41 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is T, X39 is Y | X40 is A, X41 is S, X42 is S | X51 is T |
| 021 -M 42 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is T, X39 is Y | X40 is A, X41 is A, X42 is A | X51 is T |
| 021 -M 51 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is T, X39 is Y | X40 is A, X41 is A, X42 is S | X51 is A |
| 005 /06 2/0 79/ 095 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is S, X39 is Y | X40 is A, X41 is A, X42 is S | X51 is T |
| 083 | X1 is G, X3 is T, X5 is T, X6 is Y, X7 is Y | X11 is I, X13 is P, X14 is N, X15 is S, X16 is G, X17 is G, X18 is T | X21 is V, X23 is Q, X24 is Q, X25 is G, X26 is G, X28 is Y, X33 is V | X34 is Q, X35 is S, X36 is I, X38 is T, X39 is Y | X40 is A, X41 is A, X42 is S | X51 is T |
| 088 | X1 is G, X3 is S, X5 is I, X6 is G, X7 is Y | X11 is I, X13 is P, X14 is Y, X15 is N, X16 is G, X17 is G, X18 is T | X21 is L, X23 is N, X24 is D, X25 is D, X26 is V, X28 is Y, X33 is V | X34 is Q, X35 is S, X36 is I, X38 is S, X39 is Y | X40 is A, X41 is A, X42 is S | X51 is T |
| 090 | X1 is G, X3 is T, X5 is T, X6 is D, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is V, X38 is S, X39 is Y | X40 is G, X41 is A, X42 is S | X51 is T |
| 091 | X1 is G, X3 is T, X5 is T, X6 is D, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is V, X38 is S, X39 is Y | X40 is D, X41 is A, X42 is S | X51 is T |
| 093 | X1 is G, X3 is T, X5 is T, X6 is D, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is S, X39 is Y | X40 is D, X41 is A, X42 is S | X51 is T |
| 096 | X1 is G, X3 is T, X5 is T, X6 is G, X7 is Y | X11 is I, X13 is P, X14 is N, X15 is S, X16 is G, X17 is G, X18 is T | X21 is L, X23 is N, X24 is D, X25 is D, X26 is V, X28 is Y, X33 is V | X34 is Q, X35 is S, X36 is I, X38 is S, X39 is Y | X40 is T, X41 is A, X42 is S | X51 is T |

In some embodiments, in the antibody or antigen-binding fragment thereof specifically binding to HBsAg, the VH comprises complementarity determining regions (CDR) amino acid sequences as shown below: HCDR1 as shown by SEQ ID NO: 17, HCDR2 as shown by SEQ ID NO: 21, and HCDR3 as shown by SEQ ID NO: 23, and the VL comprises LCDR1 as shown by SEQ ID NO: 25; LCDR2 as shown by SEQ ID NO: 28, and LCDR3 as shown by SEQ ID NO: 32; or
the VH comprises complementarity determining region (CDR) amino acid sequences as shown below: HCDR1 as shown by SEQ ID NO: 17, HCDR2 as shown by SEQ ID NO: 21, and HCDR3 as shown by SEQ ID NO: 23, and the VL comprises LCDR1 as shown by SEQ ID NO: 26; LCDR2 as shown by SEQ ID NO: 28, and LCDR3 as shown by SEQ ID NO: 32; or
the VH comprises complementarity determining region (CDR) amino acid sequences as shown below: HCDR1 as shown by SEQ ID NO: 20, HCDR2 as shown by SEQ ID NO: 21, and HCDR3 as shown by SEQ ID NO: 24, and the VL comprises LCDR1 as shown by SEQ ID NO: 25; LCDR2 as shown by SEQ ID NO: 28, and LCDR3 as shown by SEQ ID NO: 32; or
the VH comprises complementarity determining region (CDR) amino acid sequences as shown below: HCDR1 as shown by SEQ ID NO: 18, HCDR2 as shown by SEQ ID NO: 22, and HCDR3 as shown by SEQ ID NO: 23, and the VL comprises LCDR1 as shown by SEQ ID NO: 26; LCDR2 as shown by SEQ ID NO: 28, and LCDR3 as shown by SEQ ID NO: 32; or
the VH comprises complementarity determining region (CDR) amino acid sequences as shown below: HCDR1 as shown by SEQ ID NO: 19, HCDR2 as shown by SEQ ID NO: 21, and HCDR3 as shown by SEQ ID NO: 23, and the VL comprises LCDR1 as shown by SEQ ID NO: 27; LCDR2 as shown by SEQ ID NO: 29, and LCDR3 as shown by SEQ ID NO: 32; or
the VH comprises complementarity determining region (CDR) amino acid sequences as shown below: HCDR1 as shown by SEQ ID NO: 19, HCDR2 as shown by SEQ ID NO: 21, and HCDR3 as shown by SEQ ID NO: 23, and the VL comprises LCDR1 as shown by SEQ ID NO: 27; LCDR2 as shown by SEQ ID NO: 30, and LCDR3 as shown by SEQ ID NO: 32; or
the VH comprises complementarity determining region (CDR) amino acid sequences as shown below: HCDR1 as shown by SEQ ID NO: 19, HCDR2 as shown by SEQ ID NO: 21, and HCDR3 as shown by SEQ ID NO: 23, and the VL comprises LCDR1 as shown by SEQ ID NO: 26; LCDR2 as shown by SEQ ID NO: 30, and LCDR3 as shown by SEQ ID NO: 32; or
the VH comprises complementarity determining region (CDR) amino acid sequences as shown below: HCDR1 as shown by SEQ ID NO: 17, HCDR2 as shown by SEQ ID NO: 21, and HCDR3 as shown by SEQ ID NO: 23, and the VL comprises LCDR1 as shown by SEQ ID NO: 26; LCDR2 as shown by SEQ ID NO: 31, and LCDR3 as shown by SEQ ID NO: 32.

7. The antibody or antigen-binding fragment thereof specifically binding to HBsAg according to any one of items 1-6, comprising a human universal framework region (FR). In some embodiments, the VH comprises human subgroup III universal framework regions (FRs). In some embodiments, the VL comprises human subgroup κI universal framework. In some embodiments, a human universal framework region (FR) and one or more amino acid substitutions based on the human universal framework region (FR) are included.
In some embodiments, the framework region is a human universal framework region and comprises one or more (e.g., 1-20, 1-15, 1-10, 1-5, 1-4, 1-3) amino acids substitutions. In some embodiments, the framework region is a human universal framework region or has 70%, 80%, 90%, 95%, 97%, 98%, 99% identity to a human universal framework region. In some exemplary embodiments of the application, the VH comprises the FR amino acid sequences as shown below:
HFR1: Z1Z2Z3LZ4Z5SGAEVKKPGASZ6KVSCKAS (SEQ ID NO :39)
HFR2: Z7HWVRQAPGQGZ8EWMGW (SEQ ID NO: 40)
HFR3: NYAQKFQGRVTZ9TZ10DZ11SZ12STAYMELSZ13LRSZ14DTAVYYC (SEQ ID NO: 41)
HFR4: WGZ15GTZ16VTVSS (SEQ ID NO: 42)
VL comprises the FR amino acid sequences as shown below:
   LFR1: Z17Z18Z19LTQSPZ20Z21LSZ22SZ23GZ24RZ25TZ26Z27CRAS (SEQ ID NO: 43)
   LFR2: LZ28WYQQKPGZ29APZ30LLIZ31 (SEQ ID NO: 44)
   LFR3: Z32Z33Z34Z35GZ36PZ37RFSGSGSGTZ38FTLTIZ39SLZ40Z41Z42DZ43ATYYC (SEQ ID NO: 45)
   LFR4: FGZ44GTZ45Z46Z47IKR (SEQ ID NO: 46)
   Wherein, Z1-Z47 are each selected from any amino acid.

8. The antibody or antigen-binding fragment thereof specifically binding to HBsAg according to item 7, wherein,
Z1 is selected from Q or E, Z2 is selected from V or I, Z3 is selected from Q or T, Z4 is selected from V or K, Z5 is selected from E or Q, Z6 is selected from V or M, Z7 is selected from M, I or L, Z8 is selected from L or P, Z9 is M or I, Z10 is R or A, Z11 is T or K, Z12 is I or T, Z13 is R or S, Z14 is D or E, Z15 is selected from K or Q, Z16 is selected from L or M, Z17 is selected from D or E, Z18 is selected from I or T, Z19 is selected from Q, T or V, Z20 is selected from S, A or G, Z21 is selected from S or T, Z22 is selected from A or L, Z23 is selected from V or P, Z24 is selected from D or E, Z25 is selected from V or A, Z26 is selected from I or L, Z27 is selected from T or S, Z28 is selected from N or A, Z29 is selected from K or Q, Z30 is selected from K, Q or R, Z31 is selected from Y or S, Z32 is selected from S or N, Z33 is selected from L or R, Z34 is selected from Q or A, Z35 is selected from S or T, Z36 is selected from V or I, Z37 is selected from S or A, Z38 is selected from D or E, Z39 is S or R, Z40 is selected from Q or E, Z41 is selected from P or S, Z42 is selected from E or G, Z43 is selected from For L, Z44 is selected from G, Q or P, Z45 is selected from K or R, Z46 is selected from V or L, and Z47 is selected from D or E.
9. The antibody or antigen-binding fragment thereof specifically binding to HBsAg according to item 8, wherein:
Z1, Z2, Z3, Z4, Z5, and Z6 in HFR1 are successively selected from any combination of the following: Q, V, Q, V, E, V; E, V, Q, V, Q, V; E, V, Q, V, E, V; Q, I, T, K, E, V; or E, V, Q, V, Q, M;
Z7 and Z8 in HFR2 are successively selected from any combination of the following: M, L; L, L; I, P;
the combination of Z9, Z10, Z11, Z12, Z13, Z14 in HFR3 is: M, R, T, I, R, D; or I, A, K, T, S, E;
Z15 and Z16 in HFR4 are successively selected from any combination of the following: K, L; Q, M; Q, L; or K, M;

10. The antibody or antigen-binding fragment thereof specifically binding to HBsAg according to item 8 or 9, wherein:
Z17, Z18, Z19, Z20, Z21, Z22, Z23, Z24, Z25, Z26, and Z27 in LFR1 are successively selected from any combination of the following: D, I, Q, S, S, A, V, D, V, I, T; E, I, V, G, T, L, P, E, A, L, S; E, I, V, A, T, L, P, E, A, L, S; or E, T, T, S, T, A, V, D, V, I, T;
Z28, Z29, Z30, and Z31 in LFR2 are successively selected from any combination of the following: N, K, K, Y; A, Q, R, Y; N, K, K, S; or N, K, Q, Y;
Z32, Z33, Z34, Z35, Z36, Z37, Z38, Z39, Z40, Z41, Z42 and Z43 in LFR3 are successively selected from any combination of the following: S, L, Q, S, V, S, D, S, Q, P, E, F; S, R, A, T, I, A, E, S, Q, S, E, F; N, R, A, T, I, A, D, S, E, P, E, F; S, L, Q, S, V, S, E, R, Q, P, E, F; or S, L, Q, S, V, S, D, S, Q, P, G, L;
Z44, Z45, Z46, and Z47 in LFR4 are successively selected from any combination of the following: G, K, V, D; G, K, L, E; P, K, V, E; G, K, V, E; or Q, R, L, E.

11. The antibody or antigen-binding fragment thereof specifically binding to HBsAg according to item 10, wherein Z1-Z16 are successively selected from any combination in Table 4 below:

**Table 4:**

| Antibo dy | HFR1 | HFR2 | HFR3 | HFR4 |
|---|---|---|---|---|
| 021/07 9 | Z1 is Q, Z2 is V, Z3 is Q, Z4 is V, Z5 is E, Z6 is V | Z7 is M, Z8 is L | Z9 is M, Z10 is R, Z11 is T, Z12 is I, Z13 is R, Z14 is D | Z15 is K, Z16 is L |
| 005/09 5/096 | Z1 is E, Z2 is V, Z3 is Q, Z4 is V, Z5 is Q, Z6 is V | Z7 is M, Z8 is L | Z9 is M, Z10 is R, Z11 is T, Z12 is I, Z13 is R, Z14 is D | Z15 is Q, Z16 is M |
| 062 | Z1 is E, Z2 is V, Z3 is Q, Z4 is V, Z5 is E, Z6 is V | Z7 is M, Z8 is L | Z9 is M, Z10 is R, Z11 is T, Z12 is I, Z13 is R, Z14 is D | Z15 is Q, Z16 is L |
| 083 | Z1 is E, Z2 is V, Z3 is Q, Z4 is V, Z5 is Q, Z6 is V | Z7 is M, Z8 is L | Z9 is I, Z10 is A, Z11 is K, Z12 is T, Z13 is S, Z14 is E | Z15 is K, Z16 is L |
| 088 | Z1 is Q, Z2 is I, Z3 is T, Z4 is K, Z5 is E, Z6 is V | Z7 is L, Z8 is L | Z9 is M, Z10 is R, Z11 is T, Z12 is I, Z13 is R, Z14 is D | Z15 is K, Z16 is L |
| 090/09 1/093 | Z1 is E, Z2 is V, Z3 is Q, Z4 is V, Z5 is Q, Z6 is M | Z7 is I, Z8 is P | Z9 is M, Z10 is R, Z11 is T, Z12 is I, Z13 is R, Z14 is D | Z15 is K, Z16 is M |

12. The antibody or antigen-binding fragment thereof specifically binding to HBsAg according to any one of items 8-11, wherein Z17-Z47 are successively selected from any combination in Table 5 below:

**Table 5:**

| Antib ody | LFR1 | LFR2 | LFR3 | LFR4 |
|---|---|---|---|---|
| 021/0 83 | Z17 is D, Z18 is I, Z19 is Q, Z20 is S, Z21 is S, Z22 is A, Z23 is V, Z24 is D, Z25 is V, Z26 is I, Z27 is T | Z28 is N, Z29 is K, Z30 is K, Z31 is Y | Z32 is S, Z33 is L, Z34 is Q, Z35 is S, Z36 is V, Z37 is S, Z38 is D, Z39 is S, Z40 is Q, Z41 is P, Z42 is E, Z43 is F | Z44 is G, Z45 is K, Z46 is V, Z47 is D |
| 005/0 62/07 9/088 | Z17 is D, Z18 is I, Z19 is Q, Z20 is S, Z21 is S, Z22 is A, Z23 is V, Z24 is D, Z25 is V, Z26 is I, Z27 is T | Z28 is N, Z29 is K, Z30 is K, Z31 is Y | Z32 is S, Z33 is L, Z34 is Q, Z35 is S, Z36 is V, Z37 is S, Z38 is D, Z39 is S, Z40 is Q, Z41 is P, Z42 is E, Z43 is F | Z44 is G, Z45 is K, Z46 is L, Z47 is E |
| 090 | Z17 is E, Z18 is I, Z19 is V, Z20 is G, Z21 is T, Z22 is L, Z23 is P, Z24 is E, Z25 is A, Z26 is L, Z27 is S | Z28 is A, Z29 is Q, Z30 is R, Z31 is Y | Z32 is S, Z33 is R, Z34 is A, Z35 is T, Z36 is I, Z37 is A, Z38 is E, Z39 is S, Z40 is Q, Z41 is S, Z42 is E, Z43 is F | Z44 is P, Z45 is K, Z46 is V, Z47 is E |
| 091 | Z17 is E, Z18 is I, Z19 is V, Z20 is A, Z21 is T, Z22 is L, Z23 is P, Z24 is E, Z25 is A, Z26 is L, Z27 is S | Z28 is A, Z29 is Q, Z30 is R, Z31 is Y | Z32 is N, Z33 is R, Z34 is A, Z35 is T, Z36 is I, Z37 is A, Z38 is D, Z39 is S, Z40 is E, Z41 is P, Z42 is E, Z43 is F | Z44 is G, Z45 is K, Z46 is V, Z47 is D |
| 093 | Z17 is D, Z18 is I, Z19 is Q, Z20 is S, Z21 is S, Z22 is A, Z23 is V, Z24 is D, Z25 is V, Z26 is I, Z27 is T | Z28 is N, Z29 is K, Z30 is K, Z31 is Y | Z32 is S, Z33 is L, Z34 is Q, Z35 is S, Z36 is V, Z37 is S, Z38 is E, Z39 is R, Z40 is Q, Z41 is P, Z42 is E, Z43 is F | Z44 is G, Z45 is K, Z46 is L, Z47 is E |
| 095 | Z17 is D, Z18 is I, Z19 is Q, Z20 is S, Z21 is S, Z22 is A, Z23 is V, Z24 is D, Z25 is V, Z26 is I, Z27 is T | Z28 is N, Z29 is K, Z30 is K, Z31 is S | Z32 is S, Z33 is L, Z34 is Q, Z35 is S, Z36 is V, Z37 is S, Z38 is D, Z39 is S, Z40 is Q, Z41 is P, Z42 is G, Z43 is L | Z44 is G, Z45 is K, Z46 is V, Z47 is E |
| 096 | Z17 is E, Z18 is T, Z19 is T, Z20 is S, Z21 is T, Z22 is A, Z23 is V, Z24 is D, Z25 is V, Z26 is I, Z27 is T | Z28 is N, Z29 is K, Z30 is Q, Z31 is Y | Z32 is S, Z33 is L, Z34 is Q, Z35 is S, Z36 is V, Z37 is S, Z38 is D, Z39 is S, Z40 is Q, Z41 is P, Z42 is E, Z43 is F | Z44 is Q, Z45 is R, Z46 is L, Z47 is E |

13. The antibody or antigen-binding fragment thereof specifically binding to HBsAg according to item 8, wherein Z1-Z47 are successively selected from any combination in table 6 below:

**Table 6:**

| A ntib o d y | HFR1 | HFR 2 | HFR3 | HFR 4 | LFR1 | LFR2 | LFR3 | LFR4 |
|---|---|---|---|---|---|---|---|---|
| 0 2 1 | Z1 is Q, Z2 is V, Z3 is Q, Z4 is V, Z5 is E, Z6 is V | Z7 is M, Z8 is L | Z9 is M, Z10 is R, Z11 is T, Z12 is I, Z13 is R, Z14 is D | Z15 is K, Z16 is L | Z17 is D, Z18 is I, Z19 is Q, Z20 is S, Z21 is S, Z22 is A, Z23 is V, Z24 is D, Z25 is V, Z26 is I, Z27 is T | Z28 is N, Z29 is K, Z30 is K, Z31 is Y | Z32 is S, Z33 is L, Z34 is Q, Z35 is S, Z36 is V, Z37 is S, Z38 is D, Z39 is S, Z40 is Q, Z41 is P, Z42 is E, Z43 is F | Z44 is G, Z45 is K, Z46 is V, Z47 is D |
| 0 0 5 | Z1 is E, Z2 is V, Z3 is Q, Z4 is V, Z5 is Q, Z6 is V | Z7 is M, Z8 is L | Z9 is M, Z10 is R, Z11 is T, Z12 is I, Z13 is R, Z14 is D | Z15 is Q, Z16 is M | Z17 is D, Z18 is I, Z19 is Q, Z20 is S, Z21 is S, Z22 is A, Z23 is V, Z24 is D, Z25 is V, Z26 is I, Z27 is T | Z28 is N, Z29 is K, Z30 is K, Z31 is Y | Z32 is S, Z33 is L, Z34 is Q, Z35 is S, Z36 is V, Z37 is S, Z38 is D, Z39 is S, Z40 is Q, Z41 is P, Z42 is E, Z43 is F | Z44 is G, Z45 is K, Z46 is L, Z47 is E |
| 0 6 2 | Z1 is E, Z2 is V, Z3 is Q, Z4 is V, Z5 is E, Z6 is V | Z7 is M, Z8 is L | Z9 is M, Z10 is R, Z11 is T, Z12 is I, Z13 is R, Z14 is D | Z15 is Q, Z16 is L | Z17 is D, Z18 is I, Z19 is Q, Z20 is S, Z21 is S, Z22 is A, Z23 is V, Z24 is D, Z25 is V, Z26 is I, Z27 is T | Z28 is N, Z29 is K, Z30 is K, Z31 is Y | Z32 is S, Z33 is L, Z34 is Q, Z35 is S, Z36 is V, Z37 is S, Z38 is D, Z39 is S, Z40 is Q, Z41 is P, Z42 is E, Z43 is F | Z44 is G, Z45 is K, Z46 is L, Z47 is E |
| 0 7 9 | Z1 is Q, Z2 is V, Z3 is Q, Z4 is V, Z5 is E, Z6 is V | Z7 is M, Z8 is L | Z9 is M, Z10 is R, Z11 is T, Z12 is I, Z13 is R, Z14 is D | Z15 is K, Z16 is L | Z17 is D, Z18 is I, Z19 is Q, Z20 is S, Z21 is S, Z22 is A, Z23 is V, Z24 is D, Z25 is V, Z26 is I, Z27 is T | Z28 is N, Z29 is K, Z30 is K, Z31 is Y | Z32 is S, Z33 is L, Z34 is Q, Z35 is S, Z36 is V, Z37 is S, Z38 is D, Z39 is S, Z40 is Q, Z41 is P, Z42 is E, Z43 is F | Z44 is G, Z45 is K, Z46 is L, Z47 is E |
| 0 8 3 | Z1 is E, Z2 is V, Z3 is Q, Z4 is V, Z5 is Q, Z6 is V | Z7 is M, Z8 is L | Z9 is I, Z10 is A, Z11 is K, Z12 is T, Z13 is S, Z14 is E | Z15 is K, Z16 is L | Z17 is D, Z18 is I, Z19 is Q, Z20 is S, Z21 is S, Z22 is A, Z23 is V, Z24 is D, Z25 is V, Z26 is I, Z27 is T | Z28 is N, Z29 is K, Z30 is K, Z31 is Y | Z32 is S, Z33 is L, Z34 is Q, Z35 is S, Z36 is V, Z37 is S, Z38 is D, Z39 is S, Z40 is Q, Z41 is P, Z42 is E, Z43 is F | Z44 is G, Z45 is K, Z46 is V, Z47 is D |
| 0 8 8 | Z1 is Q, Z2 is I, Z3 is T, Z4 is K, Z5 is E, Z6 is V | Z7 is L, Z8 is L | Z9 is M, Z10 is R, Z11 is T, Z12 is I, Z13 is R, Z14 is D | Z15 is K, Z16 is L | Z17 is D, Z18 is I, Z19 is Q, Z20 is S, Z21 is S, Z22 is A, Z23 is V, Z24 is D, Z25 is V, Z26 is I, Z27 is T | Z28 is N, Z29 is K, Z30 is K, Z31 is Y | Z32 is S, Z33 is L, Z34 is Q, Z35 is S, Z36 is V, Z37 is S, Z38 is D, Z39 is S, Z40 is Q, Z41 is P, Z42 is E, Z43 is F | Z44 is G, Z45 is K, Z46 is L, Z47 is E |
| 0 9 0 | Z1 is E, Z2 is V, Z3 is Q, Z4 is V, Z5 is Q, Z6 is M | Z7 is I, Z8 is P | Z9 is M, Z10 is R, Z11 is T, Z12 is I, Z13 is R, Z14 is D | Z15 is K, Z16 is M | Z17 is E, Z18 is I, Z19 is V, Z20 is G, Z21 is T, Z22 is L, Z23 is P, Z24 is E, Z25 is A, Z26 is L, Z27 is S | Z28 is A, Z29 is Q, Z30 is R, Z31 is Y | Z32 is S, Z33 is R, Z34 is A, Z35 is T, Z36 is I, Z37 is A, Z38 is E, Z39 is S, Z40 is Q, Z41 is S, Z42 is E, Z43 is F | Z44 is P, Z45 is K, Z46 is V, Z47 is E |
| 0 9 1 | Z1 is E, Z2 is V, Z3 is Q, Z4 is V, Z5 is Q, Z6 is M | Z7 is I, Z8 is P | Z9 is M, Z10 is R, Z11 is T, Z12 is I, Z13 is R, Z14 is D | Z15 is K, Z16 is M | Z17 is E, Z18 is I, Z19 is V, Z20 is A, Z21 is T, Z22 is L, Z23 is P, Z24 is E, Z25 is A, Z26 is L, Z27 is S | Z28 is A, Z29 is Q, Z30 is R, Z31 is Y | Z32 is N, Z33 is R, Z34 is A, Z35 is T, Z36 is I, Z37 is A, Z38 is D, Z39 is S, Z40 is E, Z41 is P, Z42 is E, Z43 is F | Z44 is G, Z45 is K, Z46 is V, Z47 is D |
| 0 9 3 | Z1 is E, Z2 is V, Z3 is Q, Z4 is V, Z5 is Q, Z6 is M | Z7 is I, Z8 is P | Z9 is M, Z10 is R, Z11 is T, Z12 is I, Z13 is R, Z14 is D | Z15 is K, Z16 is M | Z17 is D, Z18 is I, Z19 is Q, Z20 is S, Z21 is S, Z22 is A, Z23 is V, Z24 is D, Z25 is V, Z26 is I, Z27 is T | Z28 is N, Z29 is K, Z30 is K, Z31 is Y | Z32 is S, Z33 is L, Z34 is Q, Z35 is S, Z36 is V, Z37 is S, Z38 is E, Z39 is R, Z40 is Q, Z41 is P, Z42 is E, Z43 is F | Z44 is G, Z45 is K, Z46 is L, Z47 is E |
| 0 9 5 | Z1 is E, Z2 is V, Z3 is Q, Z4 is V, Z5 is Q, Z6 is V | Z7 is M, Z8 is L | Z9 is M, Z10 is R, Z11 is T, Z12 is I, Z13 is R, Z14 is D | Z15 is Q, Z16 is M | Z17 is D, Z18 is I, Z19 is Q, Z20 is S, Z21 is S, Z22 is A, Z23 is V, Z24 is D, Z25 is V, Z26 is I, Z27 is T | Z28 is N, Z29 is K, Z30 is K, Z31 is S | Z32 is S, Z33 is L, Z34 is Q, Z35 is S, Z36 is V, Z37 is S, Z38 is D, Z39 is S, Z40 is Q, Z41 is P, Z42 is G, Z43 is L | Z44 is G, Z45 is K, Z46 is V, Z47 is E |
| 0 9 6 | Z1 is E, Z2 is V, Z3 is Q, Z4 is V, Z5 is Q, Z6 is V | Z7 is M, Z8 is L | Z9 is M, Z10 is R, Z11 is T, Z12 is I, Z13 is R, Z14 is D | Z15 is Q, Z16 is M | Z17 is E, Z18 is T, Z19 is T, Z20 is S, Z21 is T, Z22 is A, Z23 is V, Z24 is D, Z25 is V, Z26 is I, Z27 is T | Z28 is N, Z29 is K, Z30 is Q, Z31 is Y | Z32 is S, Z33 is L, Z34 is Q, Z35 is S, Z36 is V, Z37 is S, Z38 is D, Z39 is S, Z40 is Q, Z41 is P, Z42 is E, Z43 is F | Z44 is Q, Z45 is R, Z46 is L, Z47 is E |

14. The antibody or antigen-binding fragment thereof specifically binding to HBsAg according to item 1, wherein the VH sequence is selected from any one of SEQ ID NOs: 1-6, or amino acid sequences having at least 80%, 85%, 90%, 95%, 96%, 97%, 98%, 99% identity to any one of SEQ ID NOs: 1-6.
15. The antibody or antigen-binding fragment thereof specifically binding to HBsAg according to item 1 or 14, wherein the VL sequence is selected from any one of SEQ ID NOs: 7-13, or amino acid sequences having at least 80%, 85%, 90%, 95%, 95%, 96%, 97%, 98%, 99% identity to any one of SEQ ID NOs: 7-13.
16. The antibody or antigen-binding fragment thereof specifically binding to HBsAg according to any one of items 1-15, wherein the VH sequence and VL sequence are selected from any one group as follows:
SEQ ID NO: 1 and SEQ ID NO: 7, SEQ ID NO: 1 and SEQ ID NO: 8, SEQ ID NO: 2 and SEQ ID NO: 8, SEQ ID NO: 2 and SEQ ID NO: 12, SEQ ID NO: 2 and SEQ ID NO: 13, SEQ ID NO: 3 and SEQ ID NO: 8, SEQ ID NO: 4 and SEQ ID NO: 8, SEQ ID NO: 5 and SEQ ID NO: 9, SEQ ID NO : 5 and SEQ ID NO: 10, SEQ ID NO: 5 and SEQ ID NO: 11, SEQ ID NO: 6 and SEQ ID NO: 7, or a VH sequence and a VL sequence having at least 85% identity to the any one group.
17. The antibody or antigen-binding fragment thereof specifically binding to HBsAg according to any one of items 1-16, which is selected from Fab, F(ab')2, Fab', scFv, Fv, Fd, dAb, diabody, or multibody.
18. The antibody or antigen-binding fragment thereof specifically binding to HBsAg according to any one of items 1-17, wherein the antibody is a fully human antibody, a humanized antibody, a murine antibody, a chimeric antibody or a nanobody.
19. The antibody or antigen-binding fragment thereof specifically binding to HBsAg according to any one of items 1-18, further comprising a heavy chain constant region and a light chain constant region, wherein the heavy chain constant region is selected from a heavy chain constant region of IgG A, and the light chain constant region is a kappa chain or a lambda chain.
20. The antibody or antigen-binding fragment thereof specifically binding to HBsAg according to item 19, wherein the heavy chain constant region is that of human IgG1. In some embodiments, the heavy chain constant region comprises an amino acid sequence as shown by SEQ ID NO: 14, or comprises an amino acid sequence having 70% or more sequence identity to SEQ ID NO: 14 (e.g., amino acid sequences having 75%, 80%, 85%, 90%, 95%, 97%, 98%, 99% identity).
21. The antibody or antigen-binding fragment thereof specifically binding to HBsAg according to item 19 or 20, wherein the light chain constant region is derived from human kappa chain. In some embodiments, the light chain constant region comprises an amino acid sequence as shown by SEQ ID NO: 15, or comprises an amino acid sequence having 70% or more sequence identity (e.g., amino acid sequences having 75%, 80%, 85%, 90%, 95%, 97%, 98%, 99% identity) to SEQ ID NO: 15.
22. The antibody or antigen-binding fragment thereof specifically binding to HBsAg according to any one of items 1-21, which is a monospecific antibody, a bispecific antibody, or a multispecific antibody.
23. An isolated nucleic acid molecule, comprising a polynucleotide sequence encoding the antibody or antigen-binding fragment thereof specifically binding to HBsAg according to any one of items 1-22.
24. A construct, comprising the nucleic acid molecule according to item 23, wherein the construct is a plasmid, a phagemid, a viral vector, or a linear nucleic acid.
25. A virus, phage or cell, expressing the antibody or antigen-binding fragment thereof specifically binding to HBsAg according to any one of items 1-22, or comprising the nucleic acid molecule according to item 23 or the construct according to item 24.
26. A pharmaceutical composition, comprising the antibody or antigen-binding fragment thereof specifically binding to HBsAg according to any one of items 1-22, the nucleic acid molecule according to item 23, the construct according to item 24, or the virus, phage or cell according to item 25.
27. A kit, comprising the antibody or antigen-binding fragment thereof specifically binding to HBsAg according to any one of items 1-22, the nucleic acid molecule according to item 23, the construct according to item 24, or the virus, phage or cell according to item 25.
28. A method for preventing or treating hepatitis B virus (HBV) infection, or alleviating the symptoms of hepatitis B, comprising administering an effective amount of the pharmaceutical composition according to item 26 to a subject.
29. A method for detecting HBV, comprising contacting the antibody or antigen-binding fragment thereof specifically binding to HBsAg according to any one of items 1-22, the nucleic acid molecule according to item 23, the construct according to item 24, or the virus, phage according to item 25 with body fluid from a subject.
30. The method according to item 29, wherein the body fluid is plasma or serum.

### DESCRIPTION OF DRAWINGS

Figure 1 shows the effects of the 005, 062, 079 and 083 antibodies with murine IgG1 constant region on the plasma HBV DNA of AAV/HBV mice after tail vein injection.
Figure 2 shows the effect of 005, 062, 079 and 083 antibodies with murine IgG1 constant region on the plasma HBsAg of AAV/HBV mice after tail vein injection.
Figure 3 shows the effect of the 005, 021, 062 and 088 antibodies with murine IgG1 constant region on the plasma HBV DNA of AAV/HBV mice after tail vein injection.
Figure 4 shows the effect of 005, 021, 062 and 088 antibodies with murine IgG1 constant region on plasma HBsAg in AAV/HBV mice after tail vein injection.
Figure 5 shows the effect of tail vein injection of 021, 090, 091 and 093 antibodies with human IgG1 constant region, and intraperitoneal injection of 021 of human IgG1 constant region on plasma HBV DNA in AAV/HBV mice.
Figure 6 shows the effect of tail vein injection of 021, 090, 091 and 093 antibodies with human IgG1 constant region, and intraperitoneal injection of 021 of human IgG1 constant region on plasma HBsAg in AAV/HBV mice.

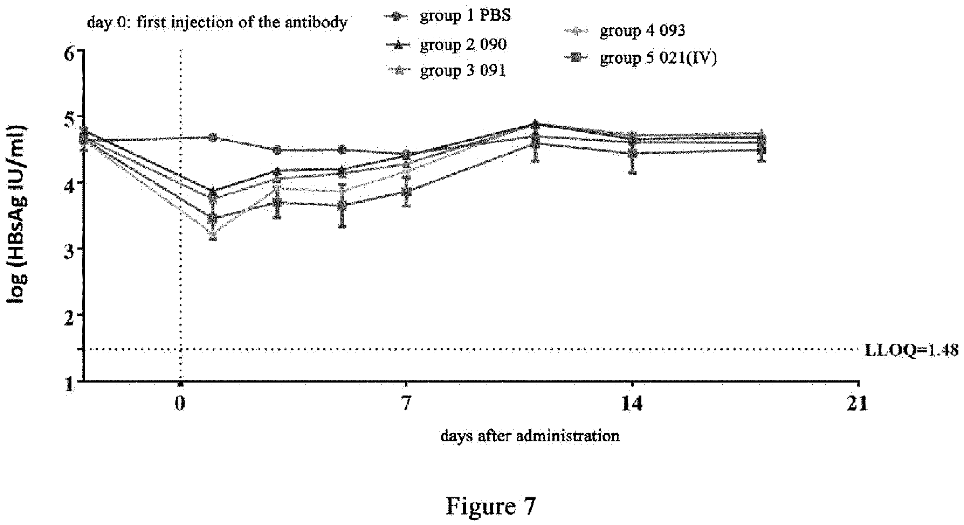

Figure 7 shows the effects of tail vein injection of 021, 095 and 096 antibodies with human IgG1 constant region, and intraperitoneal injection of 021 of human IgG1 constant region on plasma HBV DNA in AAV/HBV mice.

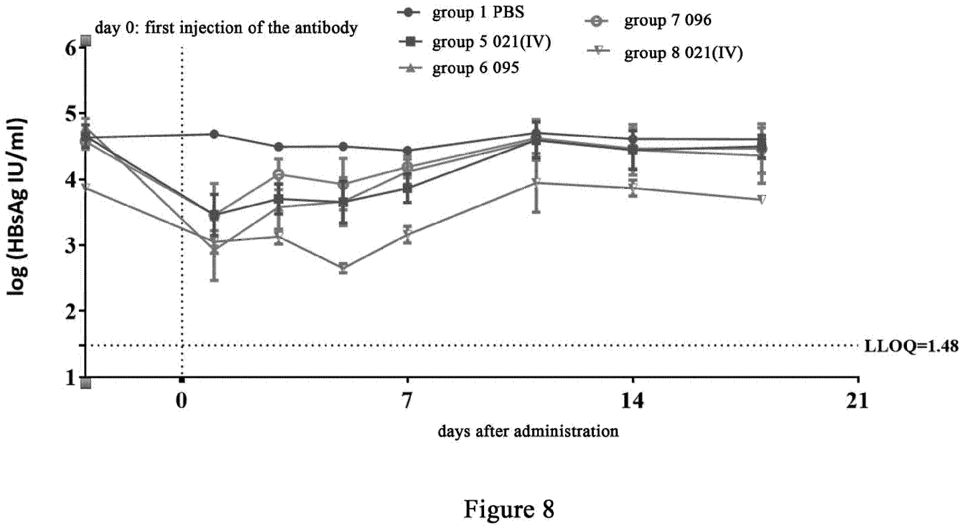

Figure 8 shows the effect of tail vein injection of 021, 095 and 096 antibodies of human IgG1 constant region and intraperitoneal injection of 021 of mouse IgG1 constant region on plasma HBsAg of AAV/HBV mice.

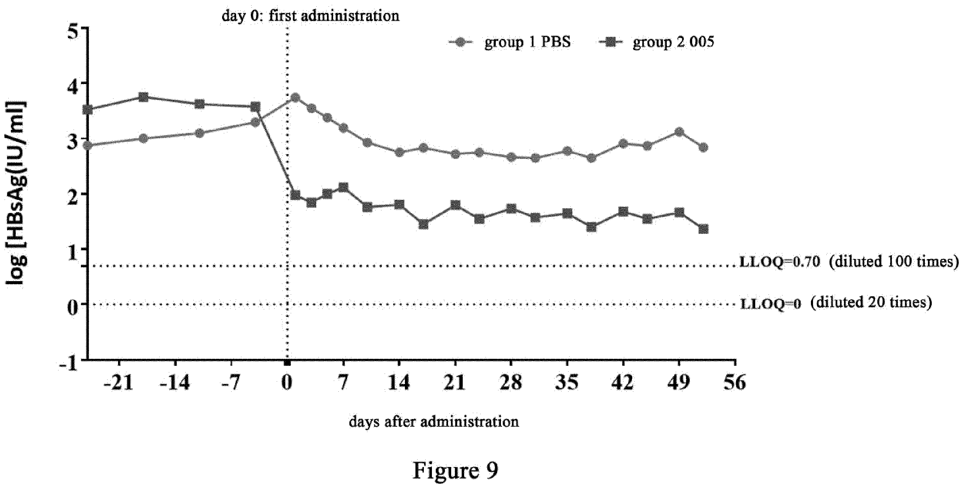

Figure 9 shows the effect of continuous administration of the antibody of the present application on plasma HBsAg of AAV/HBV mice.

### DETAILED DESCRIPTION

The purpose of this application is to provide a novel antibody or an antigen-binding fragment thereof specifically binding to hepatitis B virus surface antigen (HBsAg), which can reduce the expression level of HBsAg in the patient's blood and activate the body's own T cellular immunity, and enable the body's own immune system to effectively kill infected liver cells and inhibit the proliferation of HBV Through the specific bound antibody or its antigen-binding fragment, the present application provides new options and ideas for the prevention and treatment of HBV, and at the same time provides a new tool for the detection and diagnosis of HBV

### DEFINITION

As used herein, the term "antibody", also referred to as "immunoglobulin", encompasses antibodies having structural characteristics of native antibodies and antibody-like molecules having structural characteristics different from natural antibodies but exhibiting binding specificity to an antigenic molecule. The term antibody is intended to include immunoglobulin molecules and immunologically active fragments of immunoglobulin molecules, i.e., molecules that contain an antigen binding site. Immunoglobulin molecules can be of any type (e.g., IgG, IgE, IgM, IgD, IgA, and IgY) or subtype (e.g., IgGl, IgG2, IgG3, IgG4, IgAl, and IgA2).

As used herein, "antigen-binding fragments" of antibodies can be produced by recombinant DNA techniques or by enzymatic or chemical cleavage of intact antibodies. Antigen-binding fragments especially include Fab, Fab', F(ab')2, FV, dAb and complementarity determining region (CDR) fragments, single chain antibodies (scFv), single domain antibodies, chimeric antibodies, diabodies and such polypeptide comprising at least a portion of an immunoglobulin sufficient to confer specific antigen binding to the polypeptide. Examples of antigen antibody fragments described herein include, but are not limited to: (i) Fab fragments having VL, CL, VH and CH1 domains; (ii) Fab' fragments, i.e. Fab fragments having one or more cysteine residues at the C-terminal of the CH1 domain; (iii) Fd fragments having VH and CH1 domains; (iv) Fd' fragments having VH and CH1 domains and one or more cysteine residues at the C-terminal of the CH1 domain; (v) Fv fragments having VL and VH domains of a single arm of an antibody; (vi) dAb fragments consisting of VH domains (Ward et al., Nature 341, 544-546 (1989)); (vii) isolated CDR regions; (viii) F(ab')2 fragments, bivalent fragments comprising two Fab' fragments bridged by disulfide bonds at the hinge region; (ix) single chain antibody molecules (e.g., single chain Fv; scFv) (Bird et al., Science 242:423-426 (1988); and Huston et al., PNAS (USA) 85:5879-5883 (1988)); (x) "diabody" with two antigen-binding sites that contains a heavy chain variable region (VH) linked to a light chain variable region (VL) in the same polypeptide chain (see, e.g., EP404,097; WO93/11161; and Hollinger et al., Pr°C. Natl. Acad. Sci. USA, 90:6444-6448(1993)); (xi) "linear antibody" comprising a pair of tandem Fd segments (VH-CH1-VH-CH1), the Fd segments and the complementary light chain polypeptide together form a pair of antigen-binding domains (Zapata et al., Protein Eng. 8(10): 1057-1062 (1995); and US Patent No. 5,641,870).

The terms "heavy chain" ("CH"), "light chain" ("CL"), "light chain variable region" ("VL"), "heavy chain variable region" ("VH"), "framework region" ("FR") refers to domains in naturally occurring immunoglobulins and the corresponding domains of synthetic (e.g., recombinant) binding proteins (e.g., humanized antibodies). The basic structural unit of naturally occurring immunoglobulins, such as IgG, is a tetramer with two light chains and two heavy chains. The amino-terminal ("N") portion of each chain includes a variable region of about 100 to 110 or more amino acids primarily responsible for antigen recognition. The carboxy-terminal ("C") portion of each chain defines one constant region, light chain has a single constant region, and heavy chain typically has three constant regions and a hinge region. Thus, a naturally occurring IgG molecule has a light chain structure of N'-VL-CL-C' and an IgG heavy chain structure of N'-VH-CH1-H-CH2-CH3-C' (wherein H is the hinge region). The variable region of an IgG molecule contains complementarity determining regions (CDRs) and non-CDR fragments, the CDRs are responsible for recognizing and contacting antigens, and the non-CDR fragments (namely framework regions (FRs)) maintain the structure of the variable region and determine the position of the CDR loops. Thus, the VL and VH domains have the structure N'-FR1-CDR1-FR2-CDR2-FR3-CDR3-FR4-C'. In this application, the three CDRs of VH are called HCDR1, HCDR2, and HCDR3 respectively; the four framework regions of VH are called HFR1, HFR2, HFR3, and HFR4 respectively; the three CDRs of VL are called LCDR1, LCDR2, and LCDR3 respectively; and the four framework regions of VL are called LFR1, LFR2, LFR3, and LFR4, respectively.

In this application, the number of CDRs and FRs are determined according to the ImMunoGeneTics (IMGT) system (see, e.g., Lefranc M.P. The IMGT unique numbering for immunoglobulins, T-cell receptors, and Ig-like domains. The immunologist 7, 132-136, 1999 (1999)).

As used herein, the term "Nanobody" means a single domain antibody consisting of only the variable region of an antibody heavy chain. Because its relative molecular size is nanoscale, it is called nanobody. Nanobodies can tightly bind to antigens like normal antibodies, but they are not as easy to stick to each other and aggregate into clumps like single-chain antibodies. The term "single domain antibody" is an independent antigen-binding unit consisting of either a variable region or an engineered constant domain that only facilitates target binding. The term "diabody" is a dimer of scFv, and two scFv fragments are co-expressed by interchain pairing (cross pairing) of the VH and VL domains to form a diabody. Similarly, a multimer formed by multiple scFvs is called a multibody. For example, a triabody formed by three scFvs, a tetrabody formed by four scFvs, etc. Wherein, each scFv may have the same antigen specificity or different antigen specificity.

As used herein, "monospecific antibody", "bispecific antibody" and "multispecific antibody" respectively refer to a single antibody molecule that binds to one, two or more different antigens or one, two or more different antigen epitopes of the same antigen.

As used herein, the term "chimeric antibody" refers to an antibody that combines antibody fragments from different species. Specifically, for example, a monoclonal antibody from a species (such as a mouse), whose Fc constant region is replaced by a Fc constant region from a species (such as a human) through DNA recombination technology. See e.g., patent applications PCT/US86/02269 and EP/173,494.

As used herein, the term "humanized antibody" refers to an antibody comprising the framework regions of a human immunoglobulin and one or more CDRs from a non-human (e.g., mouse, rat, rabbit or synthetic) immunoglobulin. Except for the CDRs, all other parts of the humanized antibody are substantially identical to corresponding parts of native human immunoglobulin sequences. For methods of constructing humanized antibodies by genetic engineering, see, for example, patent application US/5,585,089.

As used herein, the term "fully humanized antibody" is intended to include antibodies having variable and constant regions derived from human germline immunoglobulin sequences. Fully humanized antibodies of the present technology may include amino acid residues not encoded by human germline immunoglobulin sequences (e.g., mutations introduced by in vitro random or site-specific mutagenesis or by in vivo somatic mutation). However, as used herein, the term "fully humanized antibody" is not intended to include antibodies in which CDR sequences derived from the germline of another mammalian species (e.g., rabbit) have been grafted onto human framework sequences. Thus, as used herein, the term "fully humanized antibody" refers to that almost every portion of its protein molecule (e.g., CDR, FR, CL, HC domains (e.g., CH1, CH2, CH3), hinge, VL, VH) is virtually non-immunogenic in humans and with only minor sequence changes or variations relative to natural human immunoglobulins. Thus, fully humanized antibodies are distinguished from chimeric or humanized antibodies. It should be noted that fully humanized antibodies can be produced by non-human animals or prokaryotic or eukaryotic cells capable of expressing functionally rearranged human immunoglobulin (e.g., heavy and/or light chain) genes.

As used herein, the term "specific binding" refers to the property of complementary binding with high affinity determined by the spatial conformation of the antigenic determinant and the variable region of the antibody molecule. This high affinity determines that once the antibody molecule binds to the antigen, it can exert its corresponding physiological function, for example, in some embodiments of the present application, the antibody binds to and helps to clear the antigen.

A "human universal framework" is a framework representing the most frequently occurring amino acid residues in a selected human immunoglobulin VL or VH framework sequence. Generally, human immunoglobulin VL or VH sequences are selected from a subgroup of variable domain sequences. Generally, a subgroup of sequences is a subgroup as in Kabat et al., Sequences of Proteins of Immunological Interest, 5th ed., NIH Publication 91-3242, Bethesda MD (1991), vol. 1-3. In some embodiments, for VL, the subgroup is subgroup κI as described by Kabat et al., supra. In some embodiments, for VH, the subgroup is subgroup III as described by Kabat et al., supra.

"Homology" or "identity" between two amino acid sequences or nucleotide sequences refers to the percentage of identical amino acid residues or nucleotide residues between the two sequences. If the two sequences to be compared to each other differ in length, sequence "homology" or "identity" preferably refers to the percentage of nucleotide residues in the shorter sequence that are identical to the amino acid residues or nucleotide residues in the longer sequence. Sequence identity can be routinely determined by using sequence analysis software commonly used in the art, such as the Wisconsin sequence analysis package.

In this application, the terms "polynucleotide" or "nucleic acid" and "nucleic acid molecule" cab be used interchangeably, including but not limited to DNA, RNA, cDNA (complementary DNA), mRNA (messenger RNA), rRNA (ribosomal RNA), shRNA (small hairpin RNA), snRNA (small nuclear RNA), snoRNA (short nucleolar RNA), miRNA (microRNA), genomic DNA, synthetic DNA, synthetic RNA and/or tRNA.

As used herein, "construct" refers to a vector (including plasmids, phagemid, viral vectors, etc.) through which a polynucleotide sequence (such as a foreign gene) can be introduced into a host cell to transform the host and facilitate expression (such as transcription and translation) of the introduced sequence.

As used herein, the term "phagemid" is a filamentous bacteriophage-derived vector whose essential components mainly include a plasmid origin of replication, a selectable marker, an intergenic region (IG region), and usually also include negative and positive strand packaging sequences and origin of replication, gene for phage coat protein, restriction endonuclease recognition site, promoter and DNA fragment encoding signal peptide. In addition, phagemids can also contain a molecular tag to facilitate screening of phagemid-based libraries. Phagemids cannot independently complete the assembly of progeny phage particles, and other structural and functional proteins required to complete their life cycle are provided by helper phages. The helper phage is a filamentous phage mutant with extremely low DNA replication efficiency. Therefore, when the helper phage and the phage formed by phagemid packaging co-infect the host bacteria, a large amount of phage containing phagemid can be packaged, while only a small amount of helper phage can be packaged.

As used herein, "plasmid" refers to DNA molecules other than chromosomes (or nucleoids) in organisms such as bacteria, yeasts, and actinomycetes, which exist in the cytoplasm or nucleus, and have the ability to replicate autonomously, enabling them to maintain a constant copy number in progeny cells and express the carried genetic information.

As used herein, "viral vector" refers to a virus-based gene vector, which is a genetically engineered viral genome that can carry foreign genes and related genetic elements, and be packaged into virus particles, and introduces the carried foreign gene into the cell through virus infection.

Unless otherwise defined herein, all technical and scientific terms used herein have the same meaning as commonly understood by one of ordinary skill in the art to which this application belongs.

### Antibodies or antigen-binding fragments thereof

In one aspect, the present application provides an antibody or an antigen-binding fragment thereof specifically binding to hepatitis B virus surface antigen (HBsAg). Typically the antibodies of the present application comprise a heavy chain variable region (VH) sequence and a light chain variable region (VL) sequence, wherein:
the heavy chain variable region comprises the general formula of complementarity determining region (CDR) amino acid sequences as shown below:
   HCDR1: X1YX3FX5X6X7Y (SEQ ID NO: 33),
   HCDR2: X11NX13X14X15X16X17X18 (SEQ ID NO: 34),
   HCDR3: ARDX21WX23X24X25X26DX28YGMDX33 (SEQ ID NO: 35);
the light chain variable region comprises the CDR amino acid sequences as shown below:
   LCDR1: X34X35X36SX38X39 (SEQ ID NO: 36),
   LCDR2: X40X41X42 (SEQ ID NO: 37),
   LCDR3: QQSYSTPLX51 (SEQ ID NO: 38);

Among them, X1, X3, X5, X6, X7, X11, X13, X14, X15, X16, X17, X18, X21, X23, X24, X25, X26, X28, X33, X34, X35, X36, X38, X39, X40, X41, X42, and X51 can all be selected from any amino acid. The any amino acid can be any of the 20 natural amino acids, and can also be an artificially synthesized or modified amino acid.

Wherein HCDR1, HCDR2, and HCDR3 represent three CDRs in the heavy chain variable region, and they are arranged sequentially from the N' terminal to the C' terminal of the heavy chain variable region. Similarly, LCDR1, LCDR2, and LCDR3 represent three CDRs in the light chain variable region, and they are arranged sequentially from the N' terminal to the C' terminal of the light chain variable region.

In the examples of the present application, some amino acids are selected to detect the CDR provided by the general formula, including: when X1 is G or A, when X3 is T, A or S, and when X5 is T, A or I, when X6 is G, A, Y or D, when X7 is Y or A, when X11 is I or A, when X13 is P or A, when X14 is N, A or Y, when X15 is S, A or N, when X16 is G or A, when X17 is G or A, when X18 is T or A, when X21 is L, A, or V, when X23 is N, Q, or A, when X24 is D, Q, or A, when X25 is D, G or A, X26 is V, G or A, X28 is Yor A, X33 is V or A, X34 is Q or A, X35 is S or A, X36 is I, A or V, X38 is T, A or S, X39 is Y or A, X40 is A, G, D, T or S, X41 is A or S, X42 is A or S, or when X51 is T or A. The detection results confirm that the antibody of the present application comprising the general formula can specifically bind to HBsAg.

Those skilled in the art should know that each value of each Xn in the general formula (wherein n represents the integer subscript of each X in the general formula) can be combined with each other. For example, in some embodiments, X1, X3, X5, X6 and X7 in HCDR1 are successively selected from any combination of the following: G, T, T, G, Y; A, T, T, G, Y; G, A, T, G, Y; G, T, A, G, Y; G, T, T, A, Y; G, T, T, G, A; G, S, I, G, Y; G, T, T, D, Y; or G, T, T, Y, Y. In some specific embodiments, HCDR1 is selected from GYTFTGYY (SEQ ID NO: 17); GYSFIGYY (SEQ ID NO: 18); GYTFTDYY (SEQ ID NO: 19); or GYTFTYYY (SEQ ID NO: 20).

X11, X13, X14, X15, X16, X17, and X18 in HCDR2 are successively selected from any combination of the following: I, P, N, S, G, G, T; A, P, N, S, G, G, T; I, A, N, S, G, G, T; I, P, A, S, G, G, T; I, P, N, A, G, G, T; I, P, N, S, A, G, T; I, P, N, S, G, A, T; I, P, N, S, G, G, A; or I, P, Y, N, G, G, T. In some specific embodiments, HCDR2 is selected from INPNSGGT (SEQ ID NO: 21); or INPYNGGT (SEQ ID NO: 22).

X21, X23, X24, X25, X26, X28, and X33 in HCDR3 are successively selected from any combination of the following: L, N, D, D, V, Y, V; A, N, D, D, V, Y, V; L, A, D, D, V, Y, V; L, N, A, D, V, Y, V; L, N, D, A, V, Y, V; L, N, D, D, A, Y, V; L, N, D, D, V, A, V; L, N, D, D, V, Y, A; or V, Q, Q, G, G, Y, V In some specific embodiments, HCDR3 is ARDLWNDDVDYYGMDV (SEQ ID NO: 23) or ARDVWQQGGYYYYMDV (SEQ ID NO: 24).

X34, X35, X36, X38, and X39 in LCDR1 are successively selected from any combination of the following: Q, S, I, T, Y; A, S, I, T, Y; Q, A, I, T, Y; Q, S, A, T, Y; Q, S, I, A, Y; Q, S, I, T, A; Q, S, I, S, Y; or Q, S, V, S, Y. In some specific embodiments, LCDR1 is selected from QSISTY (SEQ ID NO: 25); QSISSY (SEQ ID NO: 26); or QSVSSY (SEQ ID NO: 27).

X40, X41, and X42 in LCDR2 are successively selected from any combination of the following: A, A, S; S, A, S; A, S, S; A, A, A; G, A, S; D, A, S; or T, A, S; In some specific embodiments, LCDR2 is selected from AAS (SEQ ID NO: 28); GAS (SEQ ID NO: 29); DAS (SEQ ID NO: 30); or TAS (SEQ ID NO: 31).

X51 in LCDR3 is selected from: T or A. In some specific embodiments, LCDR3 is QQSYSTPLT (SEQ ID NO: 32).

In some embodiments, the VH comprises:
HCDR1 as shown by SEQ ID NO: 17, HCDR2 as shown by SEQ ID NO: 21, and HCDR3 as shown by SEQ ID NO: 23; or
HCDR1 as shown by SEQ ID NO: 20, HCDR2 as shown by SEQ ID NO: 21, and HCDR3 as shown by SEQ ID NO: 24; or
HCDR1 as shown by SEQ ID NO: 18, HCDR2 as shown by SEQ ID NO: 22, and HCDR3 as shown by SEQ ID NO: 23; or
HCDR1 as shown by SEQ ID NO: 19, HCDR2 as shown by SEQ ID NO: 21, and HCDR3 as shown by SEQ ID NO:23.

In some embodiments, the VL comprises:
LCDR1 as shown by SEQ ID NO: 25; LCDR2 as shown by SEQ ID NO: 28, and LCDR3 as shown by SEQ ID NO: 32; or
LCDR1 as shown by SEQ ID NO: 26; LCDR2 as shown by SEQ ID NO: 28, and LCDR3 as shown by SEQ ID NO: 32; or
LCDR1 as shown by SEQ ID NO: 27; LCDR2 as shown by SEQ ID NO: 29, and LCDR3 as shown by SEQ ID NO: 32; or
LCDR1 as shown by SEQ ID NO: 27; LCDR2 as shown by SEQ ID NO: 30, and LCDR3 as shown by SEQ ID NO: 32; or
LCDR1 as shown by SEQ ID NO: 26; LCDR2 as shown by SEQ ID NO: 30, and LCDR3 as shown by SEQ ID NO: 32; or
LCDR1 as shown by SEQ ID NO: 26; LCDR2 as shown by SEQ ID NO: 31, and LCDR3 as shown by SEQ ID NO: 32.

In some embodiments, the antibody or antigen-binding fragment thereof specifically binding to hepatitis B virus surface antigen (HBsAg) comprises a heavy chain variable region (VH) sequence and a light chain variable region (VL) sequence, wherein:
the VH comprises complementarity determining region (CDR) amino acid sequences as shown below: HCDR1 as shown by SEQ ID NO: 17, HCDR2 as shown by SEQ ID NO: 21, and HCDR3 as shown by SEQ ID NO: 23; and the VL comprises LCDR1 as shown by SEQ ID NO: 25, LCDR2 as shown by SEQ ID NO: 28, and LCDR3 as shown by SEQ ID NO: 32; or
the VH comprises complementarity determining region (CDR) amino acid sequences as shown below: HCDR1 as shown by SEQ ID NO: 17, HCDR2 as shown by SEQ ID NO: 21, and HCDR3 as shown by SEQ ID NO: 23; and the VL comprises LCDR1 as shown by SEQ ID NO: 26, LCDR2 as shown by SEQ ID NO: 28, and LCDR3 as shown by SEQ ID NO: 32; or
the VH comprises complementarity determining region (CDR) amino acid sequences as shown below: HCDR1 as shown by SEQ ID NO: 20, HCDR2 as shown by SEQ ID NO: 21, and HCDR3 as shown by SEQ ID NO: 24; and the VL comprises LCDR1 as shown by SEQ ID NO: 25, LCDR2 as shown by SEQ ID NO: 28, and LCDR3 as shown by SEQ ID NO: 32; or
the VH comprises complementarity determining region (CDR) amino acid sequences as shown below: HCDR1 as shown by SEQ ID NO: 18, HCDR2 as shown by SEQ ID NO: 22, and HCDR3 as shown by SEQ ID NO: 23; and the VL comprises LCDR1 as shown by SEQ ID NO: 26; LCDR2 as shown by SEQ ID NO: 28, and LCDR3 as shown by SEQ ID NO: 32; or
the VH comprises complementarity determining region (CDR) amino acid sequences as shown below: HCDR1 as shown by SEQ ID NO: 19, HCDR2 as shown by SEQ ID NO: 21, and HCDR3 as shown by SEQ ID NO: 23; and the VL comprises LCDR1 as shown by SEQ ID NO: 27, LCDR2 as shown by SEQ ID NO: 29, and LCDR3 as shown by SEQ ID NO: 32; or
the VH comprises complementarity determining region (CDR) amino acid sequences as shown below: HCDR1 as shown by SEQ ID NO: 19, HCDR2 as shown by SEQ ID NO: 21, and HCDR3 as shown by SEQ ID NO: 23; and the VL comprises LCDR1 as shown by SEQ ID NO: 27; LCDR2 as shown by SEQ ID NO: 30, and LCDR3 as shown by SEQ ID NO: 32; or
the VH comprises complementarity determining region (CDR) amino acid sequences as shown below: HCDR1 as shown by SEQ ID NO: 19, HCDR2 as shown by SEQ ID NO: 21, and HCDR3 as shown by SEQ ID NO: 23; and the VL comprises LCDR1 as shown by SEQ ID NO: 26; LCDR2 as shown by SEQ ID NO: 30, and LCDR3 as shown by SEQ ID NO: 32; or
the VH comprises complementarity determining region (CDR) amino acid sequences as shown below: HCDR1 as shown by SEQ ID NO: 17, HCDR2 as shown by SEQ ID NO: 21, and HCDR3 as shown by SEQ ID NO: 23; and the VL comprises LCDR1 as shown by SEQ ID NO: 26; LCDR2 as shown by SEQ ID NO: 31, and LCDR3 as shown by SEQ ID NO: 32.

After any combination is selected, the specific sequence of a group of CDRs can be determined according to the above general formula. The specific sequences of HCDR1, HCDR2, and HCDR3 can further form specific heavy chain CDR combinations, such as the combinations in Table 1 above. Similarly, the specific sequences of LCDR1, LCDR2, and LCDR3 can further form specific combinations of light chain CDRs, such as the combinations in Table 2 above. The HCDR group and the LCDR group can also be randomly combined. For example, in some specific embodiments, the CDR combinations of the light chain and the heavy chain are as shown by Table 3 above.

In addition, in some embodiments, the antibody specifically binding to HBsAg further comprises framework regions (FR) on both sides of each CDR region; the framework regions are human universal framework regions, or human universal framework regions comprising at least one amino acid substitution, deletion or insertion. In an exemplary embodiment, the VH comprises the FR amino acid sequences as shown below:
HFR1: Z1Z2Z3LZ4Z5SGAEVKKPGASZ6KVSCKAS (SEQ ID NO: 39)
HFR2: Z7HWVRQAPGQGZ8EWMGW (SEQ ID NO: 40)
HFR3: NYAQKFQGRVTZ9TZ10DZ11SZ12STAYMELSZ13LRSZ14DTAVYYC (SEQ ID NO: 41)
and HFR4: WGZ15GTZ16VTVSS (SEQ ID NO: 42)

VL comprises the FR amino acid sequences as shown below:
LFR1: Z17Z18Z19LTQSPZ20Z21LSZ22SZ23GZ24RZ25TZ26Z27CRAS (SEQ ID NO: 43)
LFR2: LZ28WYQQKPGZ29APZ30LLIZ31 (SEQ ID NO: 44)
LFR3: Z32Z33Z34Z35GZ36PZ37RFSGSGSGTZ38FTLTIZ39SLZ40Z41Z42DZ43ATYYC (SEQ ID NO: 45)
and LFR4: FGZ44GTZ45Z46Z47IKR (SEQ ID NO: 46), wherein Z1-Z47 are each selected from any amino acid. The any amino acid can be any of the 20 natural amino acids, and can also be an artificially synthesized or modified amino acid.

Wherein HFR1, HFR2, HFR3, and HFR4 represent 4 FRs in the heavy chain variable region, and they are arranged sequentially from the N' terminal to the C' terminal of the heavy chain variable region. Similarly, LFR1, LFR2, LFR3, and LFR4 represent the four FRs in the light chain variable region, and they are arranged sequentially from the N' terminal to the C' terminal of the light chain variable region.

In the examples of the present application, some amino acids are selected to form a specific FR sequence, and by random matching with the aforementioned CDRs, to detect the supporting effect of the general formula of the FR amino acid sequence on the CDR conformation, and the selected amino acids include, e.g.:
Z1 is selected from Q or E, Z2 is selected from V or I, Z3 is selected from Q or T, Z4 is selected from V or K, Z5 is selected from E or Q, Z6 is selected from V or M, Z7 is selected from M, I or L, Z8 is selected from L or P, Z9 is M or I, Z10 is R or A, Z11 is T or K, Z12 is I or T, Z13 is R or S, Z14 is D or E, Z15 is selected from K or Q, Z16 is selected from L or M, Z17 is selected from D or E, Z18 is selected from I or T, Z19 is selected from Q, T or V, Z20 is selected from S, A or G, Z21 is selected from S or T, Z22 is selected from A or L, Z23 is selected from V or P, Z24 is selected from D or E, Z25 is selected from V or A, Z26 is selected from I or L, Z27 is selected from T or S, Z28 is selected from N or A, Z29 is selected from K or Q, Z30 is selected from K, Q or R, Z31 is selected from Y or S, Z32 is selected from S or N, Z33 is selected from L or R, Z34 is selected from Q or A, Z35 is selected from S or T, Z36 is selected from V or I, Z37 is selected from S or A, Z38 is selected from D or E, Z39 is S or R, Z40 is selected from Q or E, Z41 is selected from P or S, Z42 is selected from E or G, Z43 is selected from For L, Z44 is selected from G, Q or P, Z45 is selected from K or R, Z46 is selected from V or L, and Z47 is selected from D or E.

In some embodiments, Z1, Z2, Z3, Z4, Z5, and Z6 in HFR1 are successively selected from any combination of the following: Q, V, Q, V, E, V; E, V, Q, V, Q, V; E, V, Q, V, E, V; Q, I, T, K, E, V; or E, V, Q, V, Q, M; Z7, Z8 in HFR2 are successively selected from any combination of the following: M, L; L, L; or I, P; the combination of Z9, Z10, Z11, Z12, Z13, Z14 in HFR3 is: M, R, T, I, R, D; or I, A, K, T, S, E; the combination of Z15 and Z16 in HFR4 is: K, L; Q, M; Q, L; or K, M. In some embodiments, Z17, Z18, Z19, Z20, Z21, Z22, Z23, Z24, Z25, Z26, and Z27 in the LFR1 are successively selected from any combination of the following: D, I, Q, S, S, A, V, D, V, I, T; E, I, V, G, T, L, P, E, A, L, S; E, I, V, A, T, L, P, E, A, L, S; or E, T, T, S, T, A, V, D, V, I, T. In some embodiments, Z28, Z29, Z30, and Z31 in LFR2 are successively selected from any combination of the following: N, K, K, Y; A, Q, R, Y; N, K, K, S; or N, K, Q, Y. In some embodiments, Z32, Z33, Z34, Z35, Z36, Z37, Z38, Z39, Z40, Z41, Z42, Z43 in LFR3 are successively selected from any combination of the following: S, L, Q, S, V, S, D, S, Q, P, E, F; S, R, A, T, I, A, E, S, Q, S, E, F; N, R, A, T, I, A, D, S, E, P, E, F; S, L, Q, S, V, S, E, R, Q, P, E, F; or S, L, Q, S, V, S, D, S, Q, P, G, L. In some embodiments, Z44, Z45, Z46, and Z47 in LFR4 are successively selected from any combination of the following: G, K, V, D; G, K, L, E; P, K, V, E; G, K, V, E; or Q, R, L, E.

In some embodiments, the variable parameter Zn of FR in the heavy chain variable region (wherein n represents the integer subscript of each Z in the general formula) can be successively selected from any combination of Table 4 above. Similarly, the variable parameter Zn of FR in the light chain variable region can be successively selected from any combination in the aforementioned Table 5. In some embodiments, the combination of CDRs of the light chain and the heavy chain are as shown by Table 6 above.

What needs to be made clear here is that all tables in this application, such as Table 1 to Table 6, all parameter values in each row of cells (such as the parameter value of Zn or the parameter value of Xn) form one or a combination.

In some specific embodiments, the VH sequence is selected from: SEQ ID NOs: 1-6. In some specific embodiments, the VL sequence is selected from: SEQ ID NOs: 7-13. In some specific embodiments, the VH sequence of the antibody is selected from: SEQ ID NOs: 1-6 and the VL sequence is selected from SEQ ID NOs: 7-13. In some specific embodiments, the VH sequence and the VL sequence are selected from selected from any one group as follows: SEQ ID NO: 1 and SEQ ID NO: 7, SEQ ID NO: 1 and SEQ ID NO: 8, SEQ ID NO: 2 and SEQ ID NO: 8, SEQ ID NO: 2 and SEQ ID NO: 12, SEQ ID NO: 2 and SEQ ID NO: 13, SEQ ID NO: 3 and SEQ ID NO: 8, SEQ ID NO: 4 and SEQ ID NO: 8, SEQ ID NO: 5 and SEQ ID NO: 9, SEQ ID NO: 5 and SEQ ID NO: 10, SEQ ID NO: 5 and SEQ ID NO: 11, or SEQ ID NO: 6 and SEQ ID NO: 7.

However, those skilled in the art shall understand that not all antibodies or antigen-binding fragments thereof comprise complete heavy and light chain variable regions. Moreover, in many cases, protein molecules that only contain antibody antigen-binding fragments still have the ability to specifically bind and/or neutralize antigens. In some embodiments, the antibody may comprise only the VH and/or VL of the antibody, such as a dAb, a single domain antibody containing only the light chain variable region, a nanobody, and the like. In some embodiments, the antibody or antigen-binding fragment thereof may be free of constant regions, such as scFvs, which comprise the aforementioned HCDRs and LCDRs. In some embodiments, the antibody or antigen-binding fragment thereof is selected from Fab, F(ab')2, Fab', Fv, Fd, diabody, or multibody. Wherein the diabody and multibody comprise the one, two or more aforementioned scFvs, and may include other scFvs that specifically bind to HBsAg or other antigens. In some embodiments, the antibody is a monospecific antibody, a bispecific antibody, or a multispecific antibody. In addition, as antigen-binding fragments of the antibodies described in the present application, various CDRs, combinations of the CDRs, and antibodies or antibody-binding fragments comprising the CDRs (such as HCDR3 and/or LCDR3, etc.) and retaining the specific recognition ability to HBsAg disclosed in the present application are also covered within the protection scope of the present application.

In some embodiments, the antibodies described herein are fully humanized antibodies, humanized antibodies, murine antibodies, chimeric antibodies or nanobodies.

In some embodiments, the antibody described herein further comprises a heavy chain constant region and a light chain constant region. In some embodiments, the heavy chain constant region and/or the light chain constant region are derived from native immunoglobulins, for example the heavy chain constant region may be derived from IgG, IgE, IgM, IgD, IgA and IgY, or subtypes, e.g., IgGl, IgG2, IgG3, IgG4, IgAl and IgA2. The light chain constant region may be selected from a kappa chain or a lambda chain. In some specific embodiments, the heavy chain constant region is the heavy chain constant region of human IgG1, comprising the amino acid sequence as shown by SEQ ID NO: 14, or comprising an amino acid sequence having 70% or more sequence identity to SEQ ID NO: 14, for example at least 70%, at least 75%, at least 80%, at least 85%, at least 88%, at least 90%, at least 92%, at least 95%, at least 96%, at least 97%, at least 98% or at least 99% sequence identity to SEQ ID NO: 14. In some specific embodiments, the light chain constant region is derived from the kappa chain, comprising the amino acid sequence as shown by SEQ ID NO: 15, or comprising an amino acid sequence having 70% or more sequence identity to SEQ ID NO: 15, for example having at least 70%, at least 75%, at least 80%, at least 85%, at least 88%, at least 90%, at least 92%, at least 95%, at least 96%, at least 97%, at least 98% or at least 99% sequence identity to SEQ ID NO: 15.

### Nucleic acid, construct, virus, phage or cell

In one aspect, the present application also provides an isolated nucleic acid molecule, comprising a polynucleotide sequence encoding the aforementioned antibody or antigen-binding fragment thereof specifically binding to HBsAg. In some embodiments, the nucleic acid molecule is an RNA or DNA molecule. In some embodiments, the nucleic acid is single-stranded or double-stranded. In some embodiments, the nucleic acid is linear or circular. The nucleic acid can be synthesized by cells or chemically. In some embodiments, the nucleic acid is a chemically modified nucleic acid molecule.

In one aspect, the present application also provides a construct comprising the aforementioned nucleic acid molecule, and the construct is plasmids, phagemids, viral vectors, or linear nucleic acids. The present application also provides a composition of constructs, comprising the aforementioned constructs, wherein each construct in the composition may respectively comprise a part of the nucleic acid molecule encoding the aforementioned antibody or antigen-binding fragment thereof specifically binding to HBsAg. For example, a composition of constructs, the constructs are plasmids, and the composition of constructs comprise plasmids respectively comprising plasmids encoding heavy chains and light chains of the aforementioned antibody molecules.

In one aspect, the present application also provides a virus, phage or cell expressing or comprising the aforementioned antibody or antigen-binding fragment thereof specifically binding to HBsAg, or a composition comprising the aforementioned nucleic acid or the aforementioned construct. The virus is selected from, for example, AAV, lentivirus and the like. The phage is selected from, for example, λ phage, T phage, M13 phage, f1 phage, fd phage and the like. The cells may be selected from, for example, mammalian cells, insect cells, bacteria, fungi, and the like.

In addition, the present application also includes use of the nucleic acid, construct, virus, phage or cell in producing the antibody or antigen-binding fragment thereof specifically binding to HBsAg.

### Pharmaceutical compositions and therapies

The present application also provides a pharmaceutical composition, comprising the aforementioned antibody or antigen-binding fragment thereof specifically binding to HBsAg, the aforementioned nucleic acid, the aforementioned construct, or the aforementioned virus, phage or cell. In some embodiments, the pharmaceutical composition further comprises an appropriate amount of pharmaceutically acceptable excipients. These agents are incorporated into formulations to improve delivery and tolerability, etc. A large number of suitable formulations can be found in a pharmacopoeia well known to all pharmaceutical chemists: Remington's Pharmaceutical Sciences, Mack Publishing Company, Easton, PA). Pharmaceutically acceptable excipients in the present application refer to non-toxic fillers, stabilizers, diluents, carriers, solvents or other preparation excipients. Among them, diluents, excipients, such as microcrystalline cellulose, mannitol, etc.; fillers, such as starch, sucrose, etc.; binders, such as starch, cellulose derivatives, alginate, gelatin and/or polyethylene pyrrolidone; disintegrants, such as calcium carbonate and/or sodium bicarbonate; absorption enhancers, such as quaternary ammonium compounds; surfactants, such as cetyl alcohol; carriers, solvents, such as water, saline, kaolin, bentonite, etc.; lubricants such as talc, calcium/magnesium stearate, polyethylene glycol, etc. In addition, the pharmaceutical composition of the present application is preferably an injection.

In some embodiments of the present application, the antibody or antigen-binding fragment thereof in the pharmaceutical composition of the present application is present at a concentration of 1 mg/ml to 1000 mg/ml, preferably at a concentration of 10 mg/ml to 1000 mg/ml, more preferably at a concentration of 50 mg/ml to 500 mg/ml, more preferably at a concentration of 100 mg/ml to 300 mg/ml.

The pharmaceutical composition of the present application preferably has a pH of 3.0 to 9.0. Among them, buffer system, preservative, surface tension agent, chelating agent, stabilizer and surfactant can be further included. In one embodiment of the application, the pharmaceutical composition of the present application is an aqueous formulation. Such formulations are usually solutions or suspensions. In a particular embodiment of the application, the pharmaceutical composition is a stable aqueous solution. In another specific embodiment of the present application, the pharmaceutical composition is a lyophilized preparation, and the physician or patient adds a solvent and/or diluent to dissolve the lyophilized preparation before use.

In one aspect, the present application also provides a method for treating HBV infection, comprising administering an appropriate dose of the aforementioned pharmaceutical composition to a patient. The appropriate dose is adjusted according to the age and body size of the subject, target disease, symptoms, administration route and the like. When the pharmaceutical composition of the present application comprises the aforementioned antibody or antigen-binding fragment thereof specifically binding to HBsAg for the treatment of various diseases and diseases associated with hepatitis B virus infection in adults, the pharmaceutical composition can be administered intravenously, typically a single dose of about 0.01 to about 20 mg of antibody per kilogram of body weight, such as about 0.1 to about 15, about 1 to about 10, about 3 to about 10 mg/kg body weight (mpk), about 12 mpk body weight. The frequency and duration of treatment may be adjusted according to the severity of the disease. For example, administering once a week to the patient.

It is known that various drug delivery systems can be used to administer the pharmaceutical composition of the present application, such as encapsulation encapsulated in liposomes, microparticles, and microcapsules, recombinant cells capable of expressing mutant viruses, receptor-mediated endocytosis (see e.g., Wu et al. (1987), J. Biol. Chem. 262:4429-4432) etc. Methods for administering the drug include, but are not limited to, intradermal, intramuscular, intraperitoneal, intravenous, subcutaneous, intranasal, epidural, and oral, etc. The pharmaceutical composition may be administered by any convenient route, such as by infusion or bolus injection, absorption through epithelial and mucosal layers (e.g. oral mucosa, rectal and small intestinal mucosa), and may be administered together with other biologically active agents. The administration method can be systemic or local.

The pharmaceutical composition can also be delivered through a sac, especially through liposome sac (see Langer (1990) Science 249: 1527-1533; Treat et al. (1989) in Liposomes in the Therapy of Infectious Disease and Cancer, Lopez Berestein and Fidler (editor), Liss, New York, pp. 353-365; Lopez-Berestein, supra, pp. 317-327)).

Under some circumstances, the pharmaceutical composition can be delivered through a controlled release system. In one embodiment, a pump can be used (see Langer, supra; Sefton (1987) CRC Crit. Ref. Biomed. Eng. 14:201). In another embodiment, polymeric materials may be used (see Medical Applications of Controlled Release, Langer and Wise (editor), CRC Press., Boca Raton, Florida (1974)). See Langer (1990) Science 249: 1527-1533 for a discussion of other controlled release systems.

The pharmaceutical composition can be administered alone or in combination, and can improve the patient's symptoms by reducing the load of hepatitis B virus surface antigen. In some embodiments, the administration is a combination administration, wherein the HBsAg antibody or antigen-binding fragment thereof is administered in combination with one or more therapeutic agents (or a second therapeutic agent). Co-administration and combination therapy are not limited to simultaneous administration, but also include regimens in which an anti-HBsAg antibody or antigen-binding fragment thereof is administered at least once in a course of treatment involving administration of at least one other therapeutic agent to the patient. The second therapeutic agent may be another HBV therapeutic agent, such as another antibody/antibody fragment, or a soluble cytokine receptor (such as interferon, interleukin, thymalfasin, etc.), nucleoside analogs (tenofovir, entecavir, adefovir, etc.).

The present application also includes use of any anti-HBsAg antibody or antigen-binding fragment described herein in the preparation of a medicament for treating a disease or condition, wherein the disease or condition is improved by reducing the level of HBsAg in a human body.

### Reagents, kits, detection methods

In one aspect, the present application provides a reagent comprising the aforementioned antibody or antigen-binding fragment thereof specifically binding to HBsAg, the complementary chain of the aforementioned nucleic acid or a fragment of the complementary chain, or the aforementioned construct, virus, phage or cell. In some embodiments, the reagent further comprises a labeling molecule. In some embodiments, the labeling molecule can be selected from enzyme labeling molecules such as horseradish peroxidase and alkaline phosphatase, fluorescent protein molecules, fluorescein molecules, biotin molecules, isotope and other imaging agents. In some embodiments, the labeling molecule is coupled to the antibody or antigen-binding fragment thereof specifically binding to HBsAg, the complementary chain of the aforementioned nucleic acid or a fragment of the complementary chain, or the aforementioned construct, virus, phage or cell to form a complex.

On the other hand, the application also provides a kit. In some embodiments, the kit comprises the aforementioned antibody or antigen-binding fragment thereof specifically binding to HBsAg. In some embodiments, the kit comprises the aforementioned nucleic acid, a fragment of the nucleic acid, or a complementary strand of the nucleic acid or a fragment of the complementary strand. In some embodiments, the kit comprises the aforementioned constructs, viruses, phage or cells. In some embodiments, the kit comprises the aforementioned reagents. In some embodiments, the kit is an in vitro detection kit. In some embodiments, the kit is an immunoassay kit, such as an ELISA kit or an immunohistochemistry kit, which comprises the aforementioned antibody or antigen-binding fragment thereof specifically binding to HBsAg. In some embodiments, the kit is an in vivo noninvasive diagnostic kit, which comprises the aforementioned antibody or antigen-binding fragment thereof specifically binding to HBsAg, and the antibody specifically binding to HBsAg or antigen-binding fragment thereof forms a complex with a marker molecule. In some embodiments, the in vivo non-invasive diagnostic kit can be used for an imaging method selected from radioimmunoimaging or targeted ultrasound contrast. When used in radioimmunoimaging, the labeling molecule is a radionuclide. When used for targeted ultrasound contrast, the marker molecule is an ultrasound contrast agent.

In addition, the present application also provides a method for detecting HBV, diagnosing HBV infection or judging the progress of HBV-related diseases by using the aforementioned antibody or antigen-binding fragment thereof specifically binding to HBsAg, the aforementioned reagent or kit.

The preferred embodiments of the present application have been described in detail above, however, the present application is not limited thereto. Within the scope of the technical concept of the present application, various simple modifications can be made to the technical solution of the present application, including the combination of various technical features in any other suitable manner, and these simple modifications and combinations should also be regarded as the content disclosed in the present application, and all belong to the protection scope of the present application.

It should be understood that the foregoing description and the following examples are intended to illustrate rather than limit the scope of the application. Other aspects within the scope of the application, advantages and modifications will be apparent to those skilled in the art to which the application pertains. Based on the embodiments of the present application, all other embodiments obtained by persons of ordinary skill in the art without making creative efforts fall within the protection scope of the present application.

### EXAMPLE

### Example 1: Construction of HBsAg antibody expression vector

The HindIII restriction site, Kozak sequence, secretion signal peptide gene and HBsAg antibody heavy chain encoding gene (including the coding gene of VH amino acid sequence and the coding gene of human IgG1constant region), the termination code and the EcoRI coding gene were sequentially fused in series, and a gene fragment was obtained by chemical synthesis. Through the EcoRI and HindIII sites, the above fragment was inserted into the eukaryotic expression plasmid pCDNA 3.1 (+) (purchased from Invitrogen Corporation, product number V790-20) and verified by sequencing to obtain the expression plasmid pCDNA3.1 (+)-DH for the heavy chain of the HBsAg antibody.

The HindIII restriction site, Kozak sequence, secretion signal peptide gene and HBsAg light chain encoding gene (including the coding gene of the VL amino acid sequence and the coding gene of the kappa chain constant region), the termination code and the EcoRI coding gene were sequentially fused in series, and a gene fragment was obtained by chemical synthesis. Through the EcoRI and HindIII sites, the above fragment was inserted into the eukaryotic expression plasmid pCDNA 3.1(+) and verified by sequencing to obtain the expression plasmid pCDNA3.1(+)-DL for the antibody light chain.

Wherein, the amino acid of the VH is: Z₁Z₂Z₃LZ₄Z₅SGAEVKKPGASZ₆KVSCKAS X₁YX₃FX₅X₆X₇YZ₇HWVRQAPGQGZ₈EWMGWX₁₁NX₁₃X₁₄X₁₅X₁₆X₁₇X₁₈NYAQKFQGRVTZ₉T Z₁₀DZ₁₁SZ₁₂STAYMELSZ₁₃LRSZ₁₄DTAVYYCARDX₂₁WX₂₃X₂₄X₂₅X₂₆DX₂₈YGMDX₃₃WGZ₁₅GT Z₁₆VTVSS (SEQ ID NO: 47);

The amino acid sequence of the VL is:

Z17Z18Z19LTQSPZ20Z21LSZ22SZ23GZ24RZ25TZ26Z27CRASX34X35X36SX38X39LZ28WYQQKPGZ29A
PZ30LLIZ31X40X41X42Z32Z33Z34Z35GZ36PZ37RFSGSGSGTZ38FTLTIZ39SLZ40Z41Z42DZ43ATYYCQ
QSYSTPLX51FGZ44GTZ45Z46Z47IKR (SEQ ID NO: 48);

Wherein the underlined part is CDR, the non underlined part is FR, Xn and Zn (n refers to any integer subscript) can be selected from any amino acid.

Among the HBsAg antibodies tested in the examples of this application, for the antibodies respectively numbered 021, 021-M1, 021-M3, 021-M5, 021-M6, 021-M7, 021-M11, 021-M13, 021-M14, 021-M15, 021-M16, 021-M17, 021-M18, 021-M21, 021-M23, 021-M24, 021-M25, 021-M26, 021-M28, 021-M33, 021-M34, 021-M35, 021-M36, 021-M38, 021-M39, 021-M40, 021-M41, 021-M42, and 021-M51, the values of Zn and Xn are respectively shown as in Table 7 (Zn) below and in Table 3 (Xn) in the summary of the application:

**Table 7:**

| F R | HFR1 | HFR 2 | HFR3 | HFR 4 | LFR1 | LFR2 | LFR3 | LFR4 |
|---|---|---|---|---|---|---|---|---|
| Zn va lu e | Z1 is Q, Z2 is V, Z3 is Q, Z4 is V, Z5 is E, Z6 is V | Z7 is M, Z8 is L | Z9 is M, Z10 is R, Z11 is T, Z12 is I, Z13 is R, Z14 is D | Z15 is K, Z16 is L | Z17 is D, Z18 is I, Z19 is Q, Z20 is S, Z21 is S, Z22 is A, Z23 is V, Z24 is D, Z25 is V, Z26 is I, Z27 is T | Z28 is N, Z29 is K, Z30 is K, Z31 is Y | Z32 is S, Z33 is L, Z34 is Q, Z35 is S, Z36 is V, Z37 is S, Z38 is D, Z39 is S, Z40 is Q, Z41 is P, Z42 is E, Z43 is F | Z44 is G, Z45 is K, Z46 is V, Z47 is D |

For antibodies whose numbers respectively numbered 005, 062, 079, 083, 088, 090, 091, 093, 095, and 096, the values of Xn and Zn are respectively as shown in Table 3 (Xn) and Table 6 (Zn) in the summary of the application. The specific VH and VL sequences are respectively:
VH sequence:
   021 and 079: 
QVQLVESGAEVKKPGASVKVSCKASGYTFTGYYMHWVRQAPGQGLEWMGWINPN
SGGTNYAQKFQGRVTMTRDTSISTAYMELSRLRSDDTAVYYCARDLWNDDVDYYGMDVW
GKGTLVTVSS (SEQ ID NO: 1)
   005, 095, and 096: 
EVQLVQSGAEVKKPGASVKVSCKASGYTFTGYYMHWVRQAPGQGLEWMGWINPNS
GGTNYAQKFQGRVTMTRDTSISTAYMELSRLRSDDTAVYYCARDLWNDDVDYYGMDVW
GQGTMVTVSS (SEQ ID NO: 2)
   062: 
EVQLVESGAEVKKPGASVKVSCKASGYTFTGYYMHWVRQAPGQGLEWMGWINPNSGGT
NYAQKFQGRVTMTRDTSISTAYMELSRLRSDDTAVYYCARDLWNDDVDYYGMDVWGQG
TLVTVSS (SEQ ID NO: 3)
   088: 
QITLKESGAEVKKPGASVKVSCKASGYSFIGYYLHWVRQAPGQGLEWMGWINPYNGGTN
YAQKFQGRVTMTRDTSISTAYMELSRLRSDDTAVYYCARDLWNDDVDYYGMDVWGKGTL
VTVSS (SEQ ID NO: 4)
   090, 091, and 093: 
EVQLVQSGAEVKKPGASMKVSCKASGYTFTDYYIHWVRQAPGQGPEWMGWINPNSG
GTNYAQKFQGRVTMTRDTSISTAYMELSRLRSDDTAVYYCARDLWNDDVDYYGMDVWG
 KGTMVTVSS (SEQ ID NO: 5)
   083: 
EVQLVQSGAEVKKPGASVKVSCKASGYTFTYYYMHWVRQAPGQGLEWMGWINPNSGGT
NYAQKFQGRVTITADKSTSTAYMELSSLRSEDTAVYYCARDVWQQGGYYYYMDVWGKGT
LVTVSS (SEQ ID NO: 6)
VL sequence:
   021, and 083: 
DIQLTQSPSSLSASVGDRVTITCRASQSISTYLNWYQQKPGKAPKLLIYAASSLQSGVPS
RFSGSGSGTDFTLTISSLQPEDFATYYCQQSYSTPLTFGGGTKVDIKR (SEQ ID NO: 7)
   005, 062, 079, and 088: 
DIQLTQSPSSLSASVGDRVTITCRASQSISSYLNWYQQKPGKAPKLLIYAASSLQSGVPSR
FSGSGSGTDFTLTISSLQPEDFATYYCQQSYSTPLTFGGGTKLEIKR (SEQ ID NO: 8)
   090: 
EIVLTQSPGTLSLSPGERATLSCRASQSVSSYLAWYQQKPGQAPRLLIYGASSRATGIPARFS
GSGSGTEFTLTISSLQSEDFATYYCQQSYSTPLTFGPGTKVEIKR (SEQ ID NO: 9)
   091: 
EIVLTQSPATLSLSPGERATLSCRASQSVSSYLAWYQQKPGQAPRLLIYDASNRATGIPARFSG
SGSGTDFTLTISSLEPEDFATYYCQQSYSTPLTFGGGTKVDIKR (SEQ ID NO: 10)
   093: 
DIQLTQSPSSLSASVGDRVTITCRASQSISSYLNWYQQKPGKAPKLLIYDASSLQSGVPSRFS
GSGSGTEFTLTIRSLQPEDFATYYCQQSYSTPLTFGGGTKLEIKR (SEQ ID NO: 11)
   095: 
DIQLTQSPSSLSASVGDRVTITCRASQSISSYLNWYQQKPGKAPKLLISAASSLQSGVPSRFS
GSGSGTDFTLTISSLQPGDLATYYCQQSYSTPLTFGGGTKVEIKR (SEQ ID NO: 12)
   096:

ETTLTQSPSTLSASVGDRVTITCRASQSISSYLNWYQQKPGKAPQLLIYTASSLQSGVPSRFS
GSGSGTDFTLTISSLQPEDFATYYCQQSYSTPLTFGQGTRLEIKR (SEQ ID NO: 13)

Among them, the corresponding relationship between the VH and VL of the above antibodies and the CDR sequences is shown in table 8 below:

**Table 8**

| Antibody name | VH | HCDR | | VL | LCDR | |
|---|---|---|---|---|---|---|
| 021 | SEQ ID NO:1 | HCDR1 | SEQ ID NO:17 | SEQ ID NO:7 | LCDR1 | SEQ ID NO:25 |
| | | HCDR2 | SEQ ID NO:21 | | LCDR2 | SEQ ID NO:28 |
| | | HCDR3 | SEQ ID NO:23 | | LCDR3 | SEQ ID NO:32 |
| 005 | SEQ ID NO:2 | HCDR1 | SEQ ID NO:17 | SEQ ID NO:8 | LCDR1 | SEQ ID NO:26 |
| | | HCDR2 | SEQ ID NO:21 | | LCDR2 | SEQ ID NO:28 |
| | | HCDR3 | SEQ ID NO:23 | | LCDR3 | SEQ ID NO:32 |
| 062 | SEQ ID NO:3 | HCDR1 | SEQ ID NO:17 | SEQ ID NO:8 | LCDR1 | SEQ ID NO:26 |
| | | HCDR2 | SEQ ID NO:21 | | LCDR2 | SEQ ID NO:28 |
| | | HCDR3 | SEQ ID NO:23 | | LCDR3 | SEQ ID NO:32 |
| 079 | SEQ ID NO:1 | HCDR1 | SEQ ID NO:17 | SEQ ID NO:8 | LCDR1 | SEQ ID NO:26 |
| | | HCDR2 | SEQ ID NO:21 | | LCDR2 | SEQ ID NO:28 |
| | | HCDR3 | SEQ ID NO:23 | | LCDR3 | SEQ ID NO:32 |
| 083 | SEQ ID NO:6 | HCDR1 | SEQ ID NO:20 | SEQ ID NO:7 | LCDR1 | SEQ ID NO:25 |
| | | HCDR2 | SEQ ID NO:21 | | LCDR2 | SEQ ID NO:28 |
| | | HCDR3 | SEQ ID NO:24 | | LCDR3 | SEQ ID NO:32 |
| 088 | SEQ ID NO:4 | HCDR1 | SEQ ID NO:18 | SEQ ID NO:8 | LCDR1 | SEQ ID NO:26 |
| | | HCDR2 | SEQ ID NO:22 | | LCDR2 | SEQ ID NO:28 |
| | | HCDR3 | SEQ ID NO:23 | | LCDR3 | SEQ ID NO:32 |
| 090 | SEQ ID NO:5 | HCDR1 | SEQ ID NO:19 | SEQ ID NO:9 | LCDR1 | SEQ ID NO:27 |
| | | HCDR2 | SEQ ID NO:21 | | LCDR2 | SEQ ID NO:29 |
| | | HCDR3 | SEQ ID NO:23 | | LCDR3 | SEQ ID NO:32 |
| 091 | SEQ ID NO:5 | HCDR1 | SEQ ID NO:19 | SEQ ID NO:10 | LCDR1 | SEQ ID NO:27 |
| | | HCDR2 | SEQ ID NO:21 | | LCDR2 | SEQ ID NO:30 |
| | | HCDR3 | SEQ ID NO:23 | | LCDR3 | SEQ ID NO:32 |
| 093 | SEQ ID NO:5 | HCDR1 | SEQ ID NO:19 | SEQ ID NO:11 | LCDR1 | SEQ ID NO:26 |
| | | HCDR2 | SEQ ID NO:21 | | LCDR2 | SEQ ID NO:30 |
| | | HCDR3 | SEQ ID NO:23 | | LCDR3 | SEQ ID NO:32 |
| 095 | SEQ ID NO:2 | HCDR1 | SEQ ID NO:17 | SEQ ID NO:12 | LCDR1 | SEQ ID NO:26 |
| | | HCDR2 | SEQ ID NO:21 | | LCDR2 | SEQ ID NO:28 |
| | | HCDR3 | SEQ ID NO:23 | | LCDR3 | SEQ ID NO:32 |
| 096 | SEQ ID NO:2 | HCDR1 | SEQ ID NO:17 | SEQ ID NO:13 | LCDR1 | SEQ ID NO:26 |
| | | HCDR2 | SEQ ID NO:21 | | LCDR2 | SEQ ID NO:31 |
| | | HCDR3 | SEQ ID NO:23 | | LCDR3 | SEQ ID NO:32 |

### Example 2: Expression and purification of anti-HBsAg antibody

Using the expression plasmids pCDNA3.1(+)-DH and pCDNA3.1(+)-DL described in Example 1, the target antibody was expressed by eukaryotic expression cells FreeStyle^{™} 293-F Cells (Invitrogen Corporation, R790-07). According to the FreeStyle^{™} 293 Expression System operating manual, the cell density was adjusted to 1×10⁶ cells/ml one day before plasmid transfection. On the day of plasmid transfection, after combining the plasmids according to the corresponding relationship between the heavy chain and the light chain (that is, combine the light chain of the antibody with the heavy chain of the antibody with the same number), they were mixed with the transfection reagent, and added to the cell culture medium. After continuous culture at 37°C and 8% CO₂ for 5-7 days, the cell culture supernatant was collected for antibody purification.

The expression supernatant was filtered with 0.22 µM filter, and the expressed antibody was captured from the expression supernatant using a Mabpurix affinity chromatography column (purchased from Sepax corporation, 65008). After equilibrating with equilibration buffer (120mM Tris+ 100mM NaCl, pH7.5), the expressed antibody was passed through the affinity chromatography column, and eluted with elution buffer (0.15M glacial acetic acid, pH2.8). The purified antibody was detected by SDS PAGE and SEC, and the purity was above 95%.

It can be seen that the amino acids selected for each Xn and each Zn in the antibodies used in the examples are different, thus these antibodies can be used to test the changes in the affinity of the antibody specific binding to antigen caused by the above-mentioned VH and VL sequence general formulas when the Xn and/or Zn parameters are changed.

### Example 3: Determination of antigen binding affinity

HBsAg protein (20 µg/ml, isolated by the applicant, comprising the amino acid sequence as shown by SEQ ID NO: 16) was used to coat the ELISA plate, 100 µl/well, sealed with a plate film, overnight at 4°C, and the plate was washed 3 times with a washing solution PBST (PBS with 0.5‰ Tween), blocked with 10% bovine serum albumin solution (prepared with washing solution PBST), adding 200 µl/well to the ELISA plate, and incubated at 37°C for 2 hours. The plate was washed 3 times with a washing solution PBST (PBS with 0.5‰ Tween), adding 100 µl/well of the gradient diluted antibody to be tested (20, 4, 0.8, 0.16, 0.032, 0.064, 0.00128, 0.00256 mg/ml, totally 8 gradients), setting a system blank control, and incubated at 37°C for 2 hours. The plate was washed 3 times with a washing solution PBST (PBS with 0.5‰ Tween), adding 100 µl/well secondary antibody (1:10000 dilution, Jackson ImmunoResearch) to the ELISA plate, and incubated at 37°C for 1 hour. Finally, the plate was washed 5 times with a washing solution PBST (PBS with 0.5‰ Tween), adding 100 µl/well TMB chromogenic solution, placing it in the dark to react for 20-40 minute at room temperature, adding equal volume of stop solution to stop the chromogenic reaction, and uing Microplate Reader to measure the absorbance (OD value) at wavelength of 450 nm. When the OD value of antigen binding is greater than 0.1, it is generally considered that binding can be detected for the antibody at the corresponding concentrationthe .

MENTTSGFLGPLLVLQAGFFLLTRILTIPQSLDSWWTSLNFLGGAPTCPGQNSQSPTSN
HSPTSCPPICPGYRWMCLRRFIIFLFILLLCLIFLLVLLDYQGMLPVCPLLPGTSTTSTGPCKTC
TIPAQGTSMFPSCCCTKPSDGNCTCIPIPSSWAFARFLWEWASVRFSWLSLLVPFVQWFVGLS
PTVWLSVIWMMWYWGPSLYNILSPFLPLLPIFFCLWVYIHHHHHH (SEQ ID NO: 16)

The HBsAg antigen-binding activity of the Microplate Reader is expressed by the absorbance (OD value) measured at wavelength of 450mn. The dilution concentrations of each antibody were 20, 4, 0.8, 0.16, 0.032, 0.0064, 0.00128, 0.000256µg/ml, totally 8 concentration gradients, the OD value detected for each antibody was less than 0.05 (usually considered to be equivalent to a blank plate, with no binding activity). When the OD value of antibody-antigen binding is greater than 0.1, it is generally considered that binding can be detected for the antibody at the corresponding concentration. The OD value of antibody 021 binding to the antigen was greater than 0.1 at the concentration of 0.16µg/ml. Based on antibody 021, its activity was defined as "++++" level; the ratio of OD value of other antibodies at a diluted concentration of 4µg/ml to OD value of antibody 021 at 4µg/ml was obtained, when the ratio was greater than 1.1, its activity was defined as "+++++" level; when the ratio was between 0.8 and 1.1, its activity was defined as "++++" level; when the ratio was between 0.6 and 0.8, its activity was defined as "+++" level; when the ratio was between 0.4 and 0.6, its activity was defined as "++ " level; when the ratio was between 0.2 and 0.4, its activity was defined as "+" level; and when the ratio was less than 0.2, it was considered there was no binding activity.

After testing, the affinity of each tested antibody is shown in Table 9 below:

**Table 9:**

| antibo dy | OD value | antibody | OD value | antibody | OD value | antibod y | OD value |
|---|---|---|---|---|---|---|---|
| 021 | ++++ | 021-M16 | ++++ | 021-M34 | ++++ | 005 | ++++ |
| 021-M1 | ++++ | 021-M17 | ++++ | 021-M35 | ++++ | 062 | ++ |
| 021-M3 | ++++ | 021-M18 | ++++ | 021-M36 | ++ | 079 | +++++ |
| 021-M5 | ++++ | 021-M21 | ++ | 021-M38 | +++++ | 083 | +++++ |
| 021-M6 | ++++ | 021-M23 | ++++++ | 021-M39 | ++ | 088 | +++++ |
| 021-M7 | +++ | 021-M24 | +++ | 021-M40 | +++++ | 090 | ++++ |
| 021-M11 | +++ | 021-M25 | +++ | 021-M41 | ++++ | 091 | ++++ |
| 021-M13 | +++ | 021-M26 | ++++ | 021-M42 | +++ | 093 | +++++ |
| 021-M14 | ++++ | 021-M28 | ++++ | 021-M51 | ++ | 095 | +++++ |
| 021-M15 | ++++ | 021-M33 | ++++ | | | 096 | +++++ |

This result shows that the antibodies of the present application have good ability to bind HBsAg specifically.

021, 021-M1, 021-M3, 021-M5, 021-M6, 021-M7, 021-M11, 021-M13, 021-M14, 021-M15, 021-M16, 021-M17, 021-M18, 021-M21, 021-M23, 021-M24, 021-M25, 021-M26, 021-M28, 021-M33, 021-M34, 021-M35, 021-M36, 021-M38, 021-M39, 021-M40, 021-M41, 021-M42, and 021-M51 have the same FR region, but the amino acids selected at the Xn position in each CDR are different, indicating that the amino acid substitution at a specific position in the CDR region of the antibody provided by the application can still make the antibodies retain the ability to specifically recognize HBsAg.

As for the antibodies respectively numbered 005, 062, 079, 083, 088, 090, 091, 093, 095, and 096, they can specifically recognize HBsAg when they have the CDRs described in this application and different FR regions, which further proves the ability of the CDR described in this application to specifically recognize and bind to the HBsAg antigen. In addition, we have also matched the appropriate FRs for the CDR regions. In the case of different Zn values, the FR can still maintain the CDR conformation of the antibody of the present application, so that the antibody can specifically recognize HBsAg.

### Example 4: drug efficacy test in vivo

In this experiment, the constant region of the antibody was selected from murine IgG1.

Animal: Male C57BL/6 mice, 5 weeks old, free from specific pathogens, were purchased from Shanghai Slack Experimental Animal Co., Ltd. and raised in independent ventilated cages. The experimental protocol approved by WuXi AppTec IACUC (IACUC#: ID01-013-2020v1.0) was used to feed the mice. The mice were injected with AAV/HBV virus (i.e., rAAV8-1.3HBV, an AAV recombinant virus containing the complete genome of D-type HBV; in this application, "AAV/HBV virus" and "rAAV8-1.3HBV" can be used interchangeably) after a 4-day environmental adaptation period.

Solvent: PBS.

Compounds tested: antibody 005, antibody 062, antibody 079, and antibody 083; dose: 12 mpk.

Recombinant rAAV8-1.3HBV: rAAV8-1.3HBV (type D, ayw) was provided by WuXi AppTec, the batch number is awy1-P4-200102, 1×10¹² viral genome (v.g.)/ml. It was diluted to 5×10¹¹ v.g./ml with sterile PBS before the experiment. Each mouse was injected with 200 µl, that is, each mouse was injected with 1×10¹¹ v.g.

Experiment method:
AAV/HBV mouse model establishment
AAV/HBV injection. rAAV8-1.3HBV was pre-prepared with sterile PBS to a solution with the concentration of 1×10¹¹ v.g./200 µl before injection. Thirty-two mice were injected with 200 µl rAAV8-1.3HBV solution through the tail vein.

Blood was collected before grouping. On days 14, 21 and 35 after virus injection, -100 µL blood was collected from the submaxillary vein for plasma collection in all infected mice. The collected venous blood was anticoagulated with K2-EDTA, centrifuged at 4°C and 7000g/min for 10 minutes to collect the plasma. The plasma was tested for HBV DNA by qPCR and for HBsAg by ELISA. The plasma samples were stored at -80°C until sent to the in vitro laboratory of WuXi AppTec Biological Department for testing related items.

The first administration was recorded as day 0, and the 45th day after infection was recorded as day 0. The mice were divided into groups according to the levels of plasma HBV DNA and HBsAg collected on days 14, 21 and 35 after virus injection, and 16 mice for the formal experiment were selected from 20 mice and randomly divided into five groups, marked as the first group to the fifth group, the number of mice in the first group was 4, and the number of mice in the remaining groups was 3. All mice were given PBS or tested antibody, 12mpk, once via caudal vein. All mice were weighed before administration.

Blood collection:
On days -1, 1, 3, 5, 7, 10, 14 and 17 after administration, blood was collected from all mice through the submandibular vein, and plasma was collected for detection of HBV DNA and HBsAg.

Experimental endpoint: test part: all mice in the test group were euthanized on day 22 after administration.

Body weight record: during the in vivo experiment, the state of the mice was observed every day, and the body weight of the mice was recorded on the day of infection, administration and blood collection.

Sample storage and transfer, all plasma samples were stored at -80°C and transferred to the in vitro laboratory of WuXi AppTec Biological Department with dry ice for corresponding testing.

The content of HBV DNA in mouse plasma was detected by quantitative PCR. The DNA in the plasma was extracted, and the experimental steps were referred to the instructions of the QIAamp 96 DNA Blood Kit. The content of HBV DNA in mouse plasma was detected by quantitative PCR. The method was briefly described as follows:
qPCR reaction mixture (Taqman Universal Master Mix (2X)) was prepared, and samples and standards were added for PCR reaction. Reaction conditions: 95°C, 10 minutes; 95°C, 15 seconds, 60°C, 1 minute, 40 cycles.

The content of HBsAg in mouse plasma was detected by ELISA. The experimental steps refer to the instructions of the HBsAg ELISA (Antu Biological, CL 0310) kit. The method was briefly described as follows: the plasma sample was diluted 2, 10, 20 or 600 times, added to the coated plate, and incubated with the enzyme conjugate (37°C, 60 minutes), repeatedly washing the plate 5 times, adding the luminescent substrate to react at room temperature in the dark for 10 minutes, and detecting the luminescence intensity with a Microplate Reader.

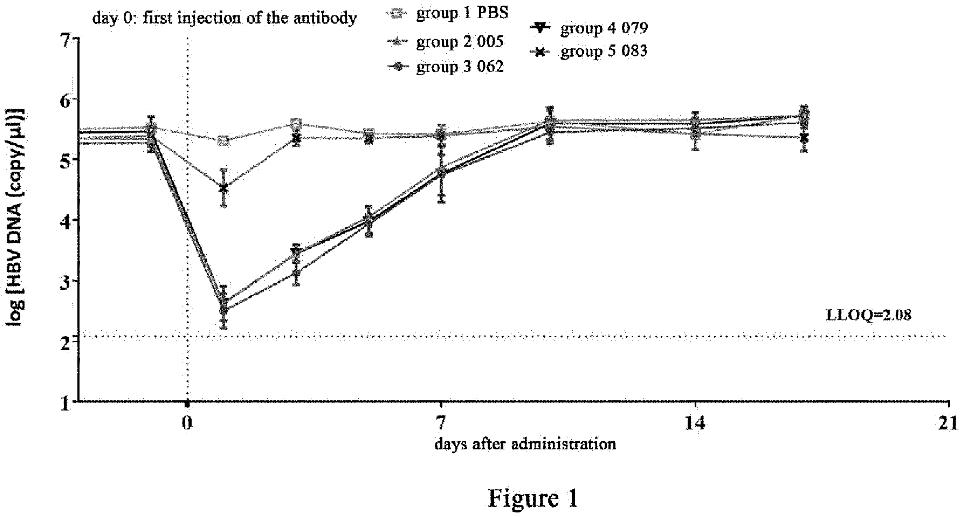

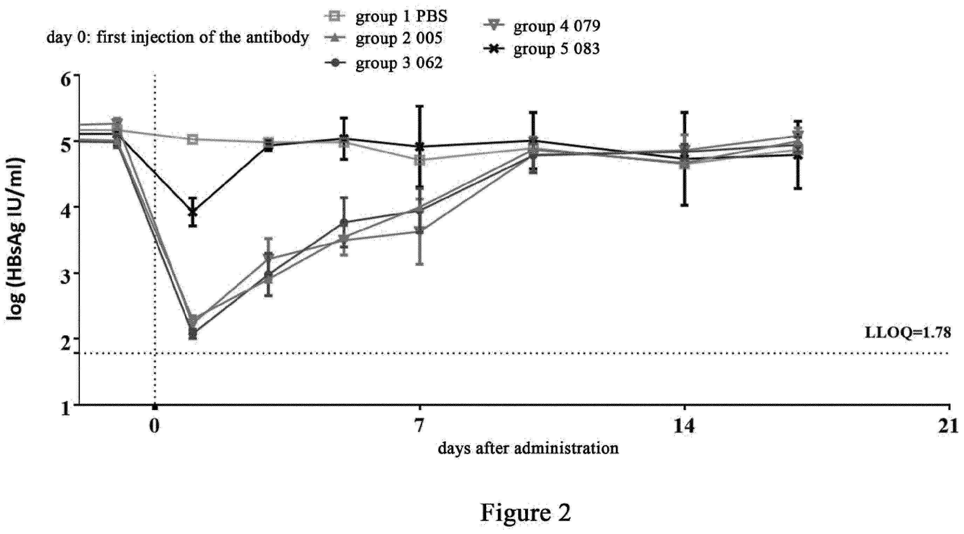

(Antibodies 005, 072, 079 and 083, dose of 12mpk) Inhibitory activity of HBV replication in the AAV/HBV mouse model was evaluated by detecting HBV DNA content and HBsAg expression in mouse plasma. The results are shown in Figure 1 and Figure 2.

The results were as follows:
1) The effect of the tested compound on the plasma HBV DNA of AAV/HBV mice
   The HBV DNA content of mice plasma in the solvent group (group 1 PBS) remained relatively stable in the experiment, fluctuating between 5.31-5.74 log10 copy/µl (fluctuation did not exceed 0.43 log10 copy/µl), compared with the solvent group, HBV DNA of the mice plasma in the treatment group 2 (antibody 005, 12mpk) decreased significantly on the first day after administration, with an average decrease of 2.69 log10 copy/µl (p<0.01), and then showed a gradual upward trend, and rose to the level of the solvent group on the 10th day, at this time HBV DNA of the mice plasma was 5.65 log10 copy/µl; the mice plasma HBV DNA in the treatment group 3 (antibody 062, 12mpk) was significantly reduced on the first day after administration, with an average decrease of 2.81 log10 copy/µl (p<0.01), then it showed a gradual upward trend, and rose to the level of the solvent group on the 10th day, at this time, HBV DNA of the mice plasma was 5.45 log10 copy/µl; HBV DNA of the mice plasma in the treatment group 4 (antibody 079, 12mpk) was significantly reduced on the first day after administration, with an average decrease of 2.68 log10 copy/µl (p<0.01), and then it showed a gradual upward trend, and rose to the level of the solvent group on the 10th day, at this time, HBV DNA of the mice plasma was 5.59 log10 copy/µl; HBV DNA of the mice plasma in the treatment group 5 (antibody 083, 12mpk) decreased significantly on the first day after administration, with an average decrease of 0.78 log10 copy/µl (p<0.01), and rose to the level of the solvent group on the third day, at this time HBV DNA of the mice plasma was 5.36 log10 copy/µl.
2) Effect of tested compound on HBsAg of AAV/HBV mouse plasma
   The HBsAg content of the mice plasma in the solvent group (group 1) remained relatively stable in the experiment, fluctuating between 4.65-5.27 log10 IU/ml (fluctuation did not exceed 0.62 log10 IU/ml); compared with the solvent group, HBsAg of the mice plasma in the treatment group 2 (antibody 005, 12mpk) decreased significantly on the first day after administration, with an average decrease of 2.73 log10 IU/ml (p<0.01), and then it showed a gradual upward trend, and rose to the level of the solvent group on the 10th day, at this time, HBsAg of the mice plasma was 4.87 log10 IU/ml; HBsAg of the mice plasma in the treatment group 3 (antibody 062, 12mpk) decreased significantly on the first day after administration, with an average decrease of 2.95 log10 IU/ml (p<0.01), then it showed a gradual upward trend, and rose to the level of the solvent group on the 10th day, at this time, HBsAg of the mice plasma was 4.78 log10 IU/ml; HBsAg of the plasma mice in the treatment group 4 (antibody 079, 12mpk) decreased significantly on the first day after administration, with an average decrease of 2.80 log10 IU/ml (p <0.01), then it showed a gradual upward trend, and rose to the level of the solvent group on the 10th day, at this time, HBsAg of the mice plasma was 4.79 log10 IU/ml; HBsAg of the mice plasma in the treatment group 5 (antibody 083, 12mpk) decreased significantly on the first day after administration, with an average decrease of 1.10 log10 IU/ml (p<0.01), and rose to the level of the solvent group on the third day, at this time, HBsAg of the mice plasma was 4.93 log10 IU/ml.

### Example 5: in vivo activity determination of antibodies 005, 021, 062 and 088

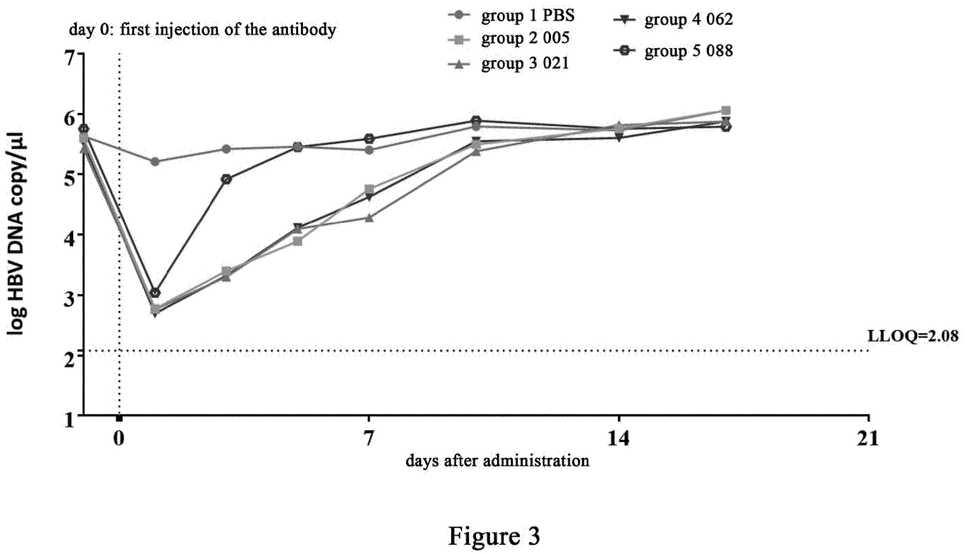

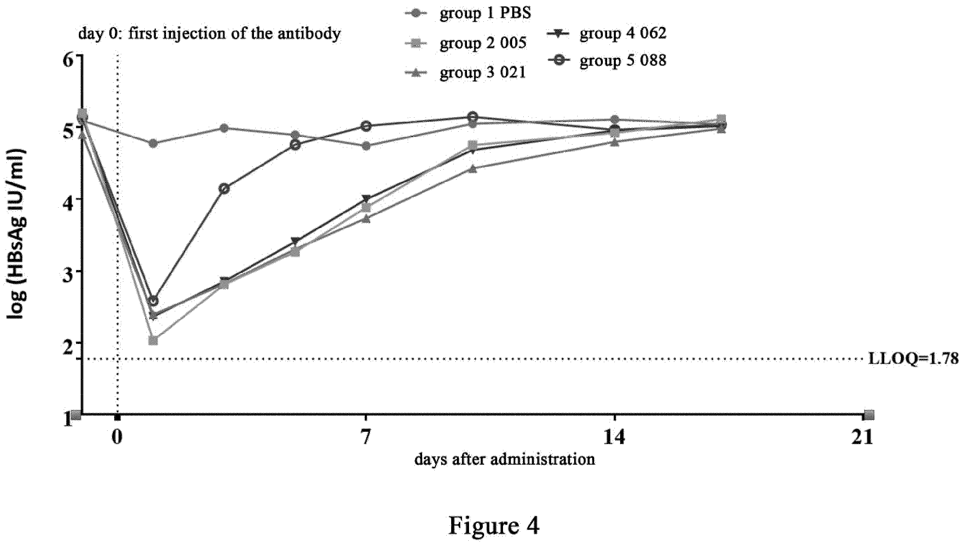

The experimental scheme was the same as that in example 4, the mice were divided into 5 groups, 4 mice in each group, and the IgG1 used was mouse IgG1. The test results are shown in Figure 3 and Figure 4. The specific results were as follows:
1) The content of HBV DNA in plasma of mice in each group.
   The HBV DNA content of the mice plasma in the solvent group (group 1) remained relatively stable in the experiment, fluctuating between 5.21-6.06 log10 copy/µl (fluctuation did not exceed 0.85 log10 copy/µl); compared with the solvent group, HBV DNA of the mice plasma in the treatment group 2 (antibody 005, 12mpk) significantly decreased on the first day after administration, with an average decrease of 2.43 log10 copy/µl (p<0.01), then showed a gradual upward trend, and rose to the level of the solvent group on the 10th day, at this time, HBV DNA of the mice plasma was 5.49 log10 copy/µl; HBV DNA of the mice plasma in the treatment group 3 (antibody 021, 12mpk) was significantly reduced on the first day after administration, with an average decrease of 2.44 log10 copy/µl (p<0.01), then showed a gradual upward trend, and rose to the level of the solvent group on the 14th day, at this time, HBV DNA of the mice plasma was 5.81 log10 copy/µl; HBV DNA of the mice plasma in the treatment group 4 (antibody 062, 12mpk) was significantly decreased on the first day after administration, with an average decrease of 2.51 log10 copy/µl (p<0.01), then it showed a gradual upward trend, rose to the level of the solvent group on the 10th day, at this time, HBV DNA of the mice plasma was 5.55 log10 copy/µl; HBV DNA of the mice plasma in the treatment group 5 ( antibody 088, 12mpk) significantly decreased on the first day after administration, with an average decrease of 2.17 log10 copy/µl (p<0.01), then showed a gradual upward trend, and rose to the level of the solvent group on the seventh day, at this time, HBV DNA of the mice plasma was 5.59 log10 copy/µl.
2) The content of plasma HBsAg in each group of mice.
   The HBsAg content of the mice plasma in the solvent group (group 1) remained relatively stable in the experiment, fluctuating between 4.74-5.11 log10 IU/ml (fluctuation did not exceed 0.37 log10 IU/ml); compared with the solvent group, HBsAg of the mice plasma in the treatment group 2 (antibody 005m, 12mpk) decreased significantly on the first day after administration, with an average decrease of 2.74 log10 IU/ml (p<0.01), then showed a gradual upward trend, and rose to the level of the solvent group on the 10th day, at this time, HBsAg of the mice plasma was 4.75 log10 IU/ml; HBsAg of the mice plasma in the treatment group 3 (antibody 021, 12mpk) decreased significantly on the first day after administration, with an average decrease of 2.38 log10 IU/ml (p<0.01), then showed a gradual upward trend, and rose to the level of the solvent group on the 17th day, at this time, HBsAg of the mice plasma was 4.98 log10 IU/ml; HBsAg of the mice plasma in the treatment group 4 (antibody 062, 12mpk) all decreased significantly on the first day after administration, with an average decrease of 2.41 and 2.60 log10 IU/ml (p<0.01), then showed a gradual upward trend, and rose to the level of the solvent group on the 14th day, at this time, HBsAg of the mice plasma was 4.95 log10 IU/ml; HBsAg of the mice plasma in the treatment group 5 (antibody 088, 12mpk) decreased significantly on the first day after administration, with an average decrease of 2.19 log10 IU/ml (p<0.01), then showed a gradual upward trend, and rose to the level of the solvent group on the fifth day, at this time, HBsAg of the mice plasma was 5.14 log10 IU/ml.

### Example 6: in vivo experiment of antibody 021 090-097

The experimental method was the same as that in example 4, the model mice were divided into 8 groups, with 4 mice in each group, and all IgG1 used were human IgG1. That is, the heavy chain and light chain constant regions of the antibody are as follows: 
ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPELLGGPS
VFLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSREEMTK
NQVSLTCLVKGFYPSDIAVEWESNGQPENNYKTTPPVLDSDGSFFLYSKLTVDKSRWQQGN
VFSCSVMHEALHNHYTQKSLSLSPGK (SEQ ID NO: 14)
 and

TVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC (SEQ ID NO: 15)

Except for group 8, which was intraperitoneally injected with antibody 021 (dose of 12mpk), mice in other groups were injected with PBS or tested antibodies (antibody 021, antibody 090, antibody 091, antibody 093, antibody 095, antibody 096, 12mpk) once through the tail vein, and the solvent group was injected with the same amount of PBS.

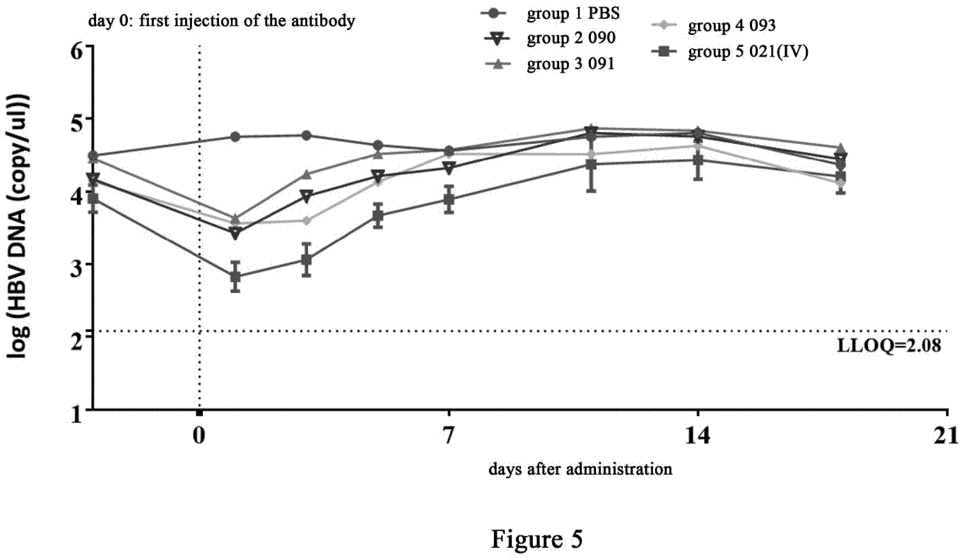

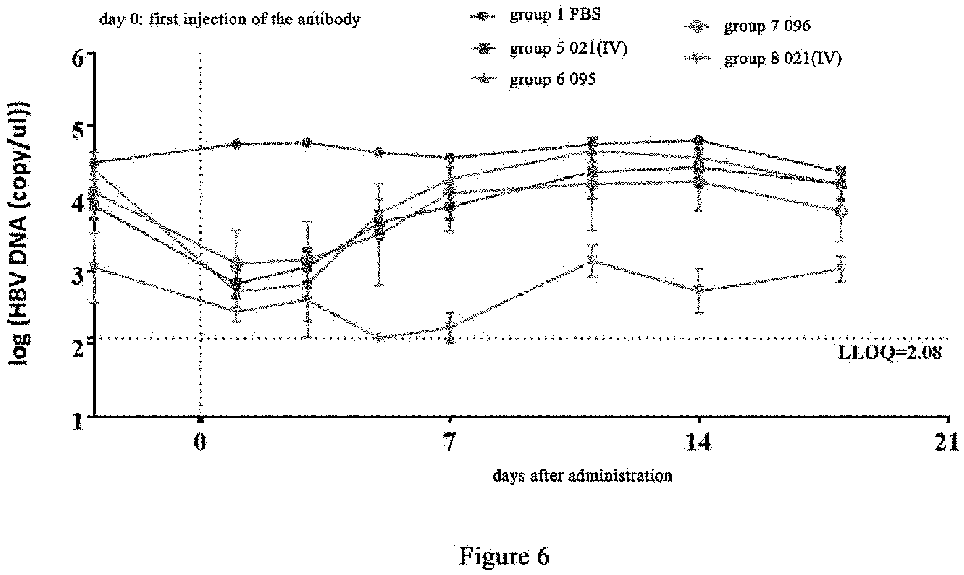

The test results are shown in Figure 5 and Figure 6. Details were as follows:
The HBV DNA and HBsAg contents of the mice plasma in the solvent group (group 1) remained relatively stable in the experiment, fluctuating between 4.36-4.80 log10 copy/µl and 4.44-4.70 log10 IU/ml, respectively (the fluctuations did not exceed 0.44 log10 copy/µl and 0.26 log10 IU/ ml); compared with the solvent group, HBV DNA and HBsAg of the mice plasma in the treatment group 2 (antibody 090, 12mpk, IV) significantly decreased on the first day after administration, with an average decrease of 1.33 log10 copy/µl (p<0.01) and 0.81 log10 IU/ml (p<0.**01)**, then showed a gradual upward trend, and rose to the level of the solvent group on the 11th and 14th days respectively, at this time, HBV DNA and HBsAg of the mice plasma were 4.80 log10 copy/µl and 4.66 log10 IU/ml respectively; HBV DNA and HBsAg of the mice plasma in the treatment group 3 (antibody 091, 12mpk, IV) significantly decreased on the first day after administration, with an average decrease of 1.12 log10 copy/µl (p<0.01) and 0.93 log10 IU/ml (p<0.01), then all showed a gradual upward trend, and rose to the level of the solvent group on the 7th and 11th days respectively, at this time, HBV DNA and HBsAg of the mice plasma were 4.57 log10 copy/µl and 4.88 log10 IU/ml respectively; HBV DNA and HBsAg of the mice plasma in the treatment group 4 (antibody 093, 12mpk, IV) significantly decreased on the first day after administration, with an average decrease of 1.19 log10 copy/µl (p<0.01) and 1.46 log10 IU/ml (p<0.01) respectively, then showed a gradual upward trend, and rose to the solvent group level on the 7th day and the 11th day, at this time, HBV DNA and HBsAg of the mice plasma were 4.52 log10 copy/µl and 4.90 log10 IU/ml respectively; HBV DNA and HBsAg of the mice plasma in the treatment group 5 (antibody 021, 12mpk, IV) decreased significantly on the first day after administration, with an average decrease of 1.93 log10 copy/µl (p<0.01) and 1.23 log10 IU/ml (p<0.01), then showed a gradual upward trend, and rose to the level of the solvent group on the 18th day and the 11th day respectively. At this time, HBV DNA and HBsAg of the mice plasma were 4.20 log10 copy/µl and 4.59 log10 IU/ml respectively; HBV DNA and HBsAg of the mice plasma in the treatment group 6 (antibody 095, 12mpk, IV) decreased significantly on the first day after administration, with an average decrease of 2.04 log10 copy/µl (p<0.01) and 1.76 log10 IU/ml (p<0.01), respectively, then showed a gradual upward trend, and both rose to the level of the solvent group on the 11th day, at this time, HBV DNA and HBsAg of the mice plasma were 4.66 log10 copy/µl and 4.59 log10 IU/ml respectively; HBV DNA and HBsAg of the mice plasma in the treatment group 7 (antibody 096, 12mpk, IV) decreased significantly on the first day after administration, with an average decrease of 1.64 log10 copy/µl (p<0.01) and 1.22 log10 IU/ml (p<0.01), then showed a gradual upward trend, and rose to the level of the solvent group on the 7th day. Then fluctuated slightly and remained until the end point of the experiment, at this time, the end point of the experiment, that is, HBV DNA of the mice plasma on the 18th day of administration was 3.83 log10 copy/µl. The mice plasma HBsAg rose to the level of the solvent group on the 11th day, and the mice plasma HBsAg was 4.62 log10 IU/ml at this time; compared with day -3, the HBV DNA content of the mice plasma in the treatment group 8 (antibody 021, 12mpk, IP) decreased by 0.60 log10 copy/µl on the first day after administration, decreased to the LLOQ (lower limit of quantification) level on the fifth day, then recovered to the level of the -3th day on the 11th day, and fluctuated between 2.73-3.14 log10 copy/µl (the fluctuation did not exceed 0.41 log10 copy/µl) between the 11th-18th day. HBsAg of the mice plasma decreased significantly on the first day after administration, with an average decrease of 0.82 log10 IU/ml, and the maximum decrease occurred on the fifth day, with an average decrease of 1.22 log10 IU/ml, then returned to the level of the -3th day on the 11th day. At this time, the mice plasma HBsAg was 3.94 log10 IU/ml.

In summary, the results of examples 4-6 show that all the antibodies in this application, including 062 with an affinity of only ++, can be used as neutralizing antibodies to HBV, significantly inhibiting the proliferation of HBV and reducing the expression of HBsAg.

### Example 7: multiple administration experiments

AAV/HBV mouse model was established as that in example 4.

The model mice were divided into two groups, namely the positive control group and the antibody 005 group (using mouse IgG1), with 4 mice in each group. The day of the first administration was counted as day 0. After that, the drug was given once every other 7 days, administered 7 times by caudal vein injection, the dose was 12mpk, and the positive control group was given 10ml/kg PBS solution.

On days -25, -18, -11, -4, 1, 3, 5, 7, 10, 14, 17, 21, 24, 28, 31, 35, 38, 42, 45, 49, and 52 after administration, all mice were taken blood through the submandibular vein, and the plasma was collected for detection of HBV DNA and HBsAg.

The results showed that the mice plasma HBsAg decreased significantly on the first day after administration, with an average decrease of 1.61 log10 IU/ml (p<0.01), and reached the lowest level on the third day, with an average decrease of about 1.73 log10 IU/ml (p<0.01). During the successive administration period, from day 0 to day 52, the HBsAg level of the mice plasma was always maintained at a low level. This result proves that the antibody of the present application can still maintain good resistance and therapeutic effect to HBV during long-term use. Moreover, injections no more than once a week can inhibit viral proliferation for a long time.

## Claims

1. An antibody or an antigen-binding fragment thereof specifically binding to hepatitis B virus surface antigen (HBsAg), comprising a heavy chain variable region (VH) sequence and a light chain variable region (VL) sequence, wherein:
the heavy chain variable region comprises complementarity determining region (CDR) amino acid sequences as shown below:
HCDR1: X1YX3FX5X6X7Y (SEQ ID NO: 33),
HCDR2: X11NX13X14X15X16X17X18 (SEQ ID NO: 34),
and HCDR3: ARDX21WX23X24X25X26DX28YGMDX33 (SEQ ID NO: 35);
the light chain variable region comprises CDR amino acid sequences as shown below:
LCDR1: X34X35X36SX38X39 (SEQ ID NO: 36),
LCDR2: X40X41X42 (SEQ ID NO: 37),
and LCDR3: QQSYSTPLX51 (SEQ ID NO: 38);
wherein:
X1 is selected from G or A, X3 is selected from T, A or S, X5 is selected from T, A or I, X6 is selected from G, A, Y or D, X7 is selected from Y or A, X11 is selected from I or A, X13 is selected from P or A, X14 is selected from N, A or Y, X15 is selected from S, A or N, X16 is selected from G or A, X17 is selected from G or A, X18 is selected from T or A, X21 is selected from L, V or A, X23 is selected from N, Q or A, X24 is selected from D, Q or A, X25 is selected from D, G or A, X26 is selected from V, G or A, X28 is selected from Y or A, X33 is selected from V or A, X34 is selected from Q or A, X35 is selected from S or A, X36 is selected from I, A or V, X38 is selected from T, A or S, X39 is selected from Y or A, X40 is selected from A, G, D, T or S, X41 is selected from A or S, X42 is selected from A or S, and X51 is selected from T or A.

2. The antibody or antigen-binding fragment thereof specifically binding to HBsAg according to claim 1, wherein the VH comprises complementarity determining region (CDR) amino acid sequences as shown below: HCDR1 as shown by SEQ ID NO: 17, HCDR2 as shown by SEQ ID NO: 21, and HCDR3 as shown by SEQ ID NO: 23, and the VL comprises LCDR1 as shown by SEQ ID NO: 25; LCDR2 as shown by SEQ ID NO: 28, and LCDR3 as shown by SEQ ID NO: 32; or
the VH comprises complementarity determining region (CDR) amino acid sequences as shown below: HCDR1 as shown by SEQ ID NO: 17, HCDR2 as shown by SEQ ID NO: 21, and HCDR3 as shown by SEQ ID NO: 23, and the VL comprises LCDR1 as shown by SEQ ID NO: 26; LCDR2 as shown by SEQ ID NO: 28, and LCDR3 as shown by SEQ ID NO: 32; or
the VH comprises complementarity determining region (CDR) amino acid sequences as shown below: HCDR1 as shown by SEQ ID NO: 20, HCDR2 as shown by SEQ ID NO: 21, and HCDR3 as shown by SEQ ID NO: 24, and the VL comprises LCDR1 as shown by SEQ ID NO: 25; LCDR2 as shown by SEQ ID NO: 28, and LCDR3 as shown by SEQ ID NO: 32; or
the VH comprises complementarity determining region (CDR) amino acid sequences as shown below: HCDR1 as shown by SEQ ID NO: 18, HCDR2 as shown by SEQ ID NO: 22, and HCDR3 as shown by SEQ ID NO: 23, and the VL comprises LCDR1 as shown by SEQ ID NO: 26; LCDR2 as shown by SEQ ID NO: 28, and LCDR3 as shown by SEQ ID NO: 32; or
the VH comprises complementarity determining region (CDR) amino acid sequences as shown below: HCDR1 as shown by SEQ ID NO: 19, HCDR2 as shown by SEQ ID NO: 21, and HCDR3 as shown by SEQ ID NO: 23, and the VL comprises LCDR1 as shown by SEQ ID NO: 27; LCDR2 as shown by SEQ ID NO: 29, and LCDR3 as shown by SEQ ID NO: 32; or
the VH comprises complementarity determining region (CDR) amino acid sequences as shown below: HCDR1 as shown by SEQ ID NO: 19, HCDR2 as shown by SEQ ID NO: 21, and HCDR3 as shown by SEQ ID NO: 23, and the VL comprises LCDR1 as shown by SEQ ID NO: 27; LCDR2 as shown by SEQ ID NO: 30, and LCDR3 as shown by SEQ ID NO: 32; or
the VH comprises complementarity determining region (CDR) amino acid sequences as shown below: HCDR1 as shown by SEQ ID NO: 19, HCDR2 as shown by SEQ ID NO: 21, and HCDR3 as shown by SEQ ID NO: 23, and the VL comprises LCDR1 as shown by SEQ ID NO: 26; LCDR2 as shown by SEQ ID NO: 30, and LCDR3 as shown by SEQ ID NO: 32; or
the VH comprises complementarity determining region (CDR) amino acid sequences as shown below: HCDR1 as shown by SEQ ID NO: 17, HCDR2 as shown by SEQ ID NO: 21, and HCDR3 as shown by SEQ ID NO: 23, and the VL comprises LCDR1 as shown by SEQ ID NO:26; LCDR2 as shown by SEQ ID NO:31, and LCDR3 as shown by SEQ ID NO:32.

3. The antibody or antigen-binding fragment thereof specifically binding to HBsAg according to claim 1 or 2, comprising a human universal framework region (FR).

4. The antibody or antigen-binding fragment thereof specifically binding to HBsAg according to any one of claims 1-3, wherein,
VH comprises FR amino acid sequences as shown below:
HFR1: Z1Z2Z3LZ4Z5SGAEVKKPGASZ6KVSCKAS (SEQ ID NO: 39)
HFR2: Z7HWVRQAPGQGZ8EWMGW (SEQ ID NO: 40)
HFR3: NYAQKFQGRVTZ9TZ10DZ11SZ12STAYMELSZ13LRSZ14DTAVYYC (SEQ ID NO: 41)
and HFR4: WGZ15GTZ16VTVSS (SEQ ID NO: 42)
VL comprises FR amino acid sequences as shown below:
LFR1: Z17Z18Z19LTQSPZ20Z21LSZ22SZ23GZ24RZ25TZ26Z27CRAS (SEQ ID NO: 43)
LFR2: LZ28WYQQKPGZ29APZ30LLIZ31 (SEQ ID NO: 44)
LFR3: Z32Z33Z34Z35GZ36PZ37RFSGSGSGTZ38FTLTIZ39SLZ40Z41Z42DZ43ATYYC (SEQ ID NO: 45)
and LFR4: FGZ44GTZ45Z46Z47IKR (SEQ ID NO: 46)
wherein,
Z1 is selected from Q or E, Z2 is selected from V or I, Z3 is selected from Q or T, Z4 is selected from V or K, Z5 is selected from E or Q, Z6 is selected from V or M, Z7 is selected from M, I or L, Z8 is selected from L or P, Z9 is M or I, Z10 is R or A, Z11 is T or K, Z12 is I or T, Z13 is R or S, Z14 is D or E, Z15 is selected from K or Q, Z16 is selected from L or M, Z17 is selected from D or E, Z18 is selected from I or T, Z19 is selected from Q, T or V, Z20 is selected from S, A or G, Z21 is selected from S or T, Z22 is selected from A or L, Z23 is selected from V or P, Z24 is selected from D or E, Z25 is selected from V or A, Z26 is selected from I or L, Z27 is selected from T or S, Z28 is selected from N or A, Z29 is selected from K or Q, Z30 is selected from K, Q or R, Z31 is selected from Y or S, Z32 is selected from S or N, Z33 is selected from L or R, Z34 is selected from Q or A, Z35 is selected from S or T, Z36 is selected from V or I, Z37 is selected from S or A, Z38 is selected from D or E, Z39 is S or R, Z40 is selected from Q or E, Z41 is selected from P or S, Z42 is selected from E or G, Z43 is selected from F or L, Z44 is selected from G, Q or P, Z45 is selected from K or R, Z46 is selected from V or L, and Z47 is selected from D or E.

5. The antibody or antigen-binding fragment thereof specifically binding to HBsAg according to any one of claims 1-4, wherein the VH sequence is selected from any one of SEQ ID NOs: 1-6, or amino acid sequence having at least 85% identity to any one of SEQ ID NOs: 1-6.

6. The antibody or antigen-binding fragment thereof specifically binding to HBsAg according to any one of claims 1-5, wherein the VL sequence is selected from any one of SEQ ID NOs: 7-13, or amino acid sequences having at least 85% identity to any one of SEQ ID NOs: 7-13.

7. The antibody or antigen-binding fragment thereof specifically binding to HBsAg according to any one of claims 1-6, wherein the VH sequence and the VL sequence are selected from any one group as follows: SEQ ID NO: 1 and SEQ ID NO: 7, SEQ ID NO: 1 and SEQ ID NO: 8, SEQ ID NO: 2 and SEQ ID NO: 8, SEQ ID NO: 2 and SEQ ID NO: 12, SEQ ID NO: 2 and SEQ ID NO: 13, SEQ ID NO : 3 and SEQ ID NO: 8, SEQ ID NO: 4 and SEQ ID NO: 8, SEQ ID NO: 5 and SEQ ID NO: 9, SEQ ID NO: 5 and SEQ ID NO: 10, SEQ ID NO: 5 and SEQ ID NO: 11, SEQ ID NO: 6 and SEQ ID NO: 7, or a VH sequence and a VL sequence having at least 85% identity to the any one group.

8. The antibody or antigen-binding fragment thereof specifically binding to HBsAg according to any one of claims 1-7, which is Fab, F(ab')2, Fab', scFv, Fv, Fd, dAb, diabody, or multibody.

9. The antibody or antigen-binding fragment thereof specifically binding to HBsAg according to any one of claims 1-8, wherein the antibody is a fully human antibody, a humanized antibody, a murine antibody, a chimeric antibody or a nanobody.

10. The antibody or antigen-binding fragment thereof specifically binding to HBsAg according to any one of claims 1-9, which is a monospecific antibody, a bispecific antibody, or a multispecific antibody.

11. The antibody or antigen-binding fragment thereof specifically binding to HBsAg according to any one of claims 1-10, further comprising a heavy chain constant region and a light chain constant region, wherein the heavy chain constant region is a heavy chain constant region of IgG A, and the light chain constant region is a kappa chain or a lambda chain.

12. An isolated nucleic acid molecule comprising a polynucleotide sequence encoding the antibody or antigen-binding fragment thereof specifically binding to HBsAg according to any one of claims 1-11.

13. A construct comprising the nucleic acid molecule according to claim 12.

14. The construct according to claim 13, wherein the construct is a plasmid, a phagemid, a viral vector, or a linear nucleic acid.

15. A virus, phage or cell expressing the antibody or antigen-binding fragment thereof specifically binding to HBsAg according to any one of claims 1-11, or comprising the nucleic acid molecule according to claim 12 or the construct according to claim 13 or 14.

16. A pharmaceutical composition, comprising the antibody or antigen-binding fragment thereof specifically binding to HBsAg according to any one of claims 1-11, the nucleic acid molecule according to claim 12, the construct according to claim 13 or 14, or the virus, phage or cell according to claim 15.

17. A kit, comprising the antibody or antigen-binding fragment thereof specifically binding to HBsAg according to any one of claims 1-11, the nucleic acid molecule according to claim 12, the construct according to claim 13 or 14, or the virus, phage or cell according to claim 15.

18. A method for preventing or treating hepatitis B virus (HBV) infection, or alleviating the symptoms of hepatitis B, comprising administering an effective amount of the pharmaceutical composition according to claim 16 to a subject.

19. A method for detecting HBV, comprising contacting the antibody or antigen-binding fragment thereof specifically binding to HBsAg according to any one of claims 1-11, the nucleic acid molecule according to claim 12, the construct according to claim 13 or 14, or the virus, phage or cell according to claim 15 with body fluid from a subject.

20. The method according to claim 19, wherein the body fluid from the subject is plasma or serum.
